(19) Europäisches Patentamt / European Patent Office / Office européen des brevets

(11) **EP 4 012 417 B1**

(12) **EUROPEAN PATENT SPECIFICATION**

(45) Date of publication and mention
of the grant of the patent:
**06.05.2026  Bulletin 2026/19**

(21) Application number: **20849959.0**

(22) Date of filing: **17.03.2020**

(51) International Patent Classification (IPC):
*G01N 35/00* (2006.01)     *G16H 10/40* (2018.01)
*G16H 40/20* (2018.01)     *G16H 40/67* (2018.01)

(52) Cooperative Patent Classification (CPC):
**G01N 35/00; G16H 10/40; G16H 40/20;
G16H 40/67;** G01N 2035/00326; G01N 2035/009

(86) International application number:
**PCT/JP2020/011662**

(87) International publication number:
**WO 2021/024539 (11.02.2021 Gazette 2021/06)**

(54) **AUTOMATIC ANALYSIS SYSTEM AND ALARM HANDLING METHOD**

AUTOMATISCHES ANALYSESYSTEM UND VERFAHREN ZUR ALARMHANDHABUNG

SYSTÈME D'ANALYSE AUTOMATIQUE ET PROCÉDÉ DE MANIPULATION D'ALARME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.08.2019   JP 2019145362**

(43) Date of publication of application:
**15.06.2022  Bulletin 2022/24**

(73) Proprietor: **Hitachi High-Tech Corporation**
**Minato-ku**
**Tokyo 105-6409 (JP)**

(72) Inventors:
• **SEKI, Yoshihiro**
  **Tokyo 105-6409 (JP)**
• **KAWAI, Tomoakira**
  **Tokyo 100-8280 (JP)**
• **YANO, Shigeru**
  **Tokyo 105-6409 (JP)**
• **FUKAMI, Misato**
  **Tokyo 100-8280 (JP)**
• **MASUDA, Ai**
  **Tokyo 100-8280 (JP)**

(74) Representative: **Strehl & Partner mbB**
**Maximilianstrasse 54**
**80538 München (DE)**

(56) References cited:
EP-A1- 2 775 307     JP-A- 2011 064 682
JP-A- 2014 194 401     JP-A- 2016 024 194
JP-A- H07 260 793     US-A1- 2019 079 107

## Description

Technical Field

The present invention relates to an automatic analysis system including an automatic analyzer or the like and an alarm handling method for analyzing an analyte derived from a biological sample such as blood or urine.

Background Art

An automatic analyzer that analyzes a biological sample such as blood or urine has merits such as high reproducibility of analysis accuracy and a high analysis processing speed, and thus is indispensable for current diagnosis. There are a plurality of types of such automatic analyzer depending on the analysis type. Examples include a colorimetric analyzer that performs biochemical analysis, an immunoanalyzer that analyzes an antigen or antibody in a sample using an antigen-antibody reaction, a coagulation analyzer that measures coagulability of blood, a blood cell counter that measures the number of blood cell components in blood, and the like. When analyzing a sample by the automatic analyzer, a pre-processing device that performs pre-processing such as centrifugation of blood or dispensing of the sample to prepare a plurality of child analytes is also used. In a hospital, inspection center, and the like, it is common to use a plurality of such automatic analyzers and sample pre-processing devices.

When an error occurs in such an automatic analyzer or sample pre-processing device, analysis may be stopped and a diagnosis made by a doctor may be delayed if the error is not immediately handled. Examples of the error include reagent replacement and replacement of a consumable such a lamp. PTL 1 discloses a technique of, when an error occurs, notifying a person-in-charge of the error and sharing who handles the error, based on information that registers errors that can be handled by the person-in-charge. PTL 2 describes a technique of, when an error occurs, notifying a person-in-charge of the error based on information that registers errors that can be handled by the person-in-charge, position information of the person-in-charge and a device generating the error, and schedule information of the person-in-charge which is used to confirm whether the person-in-charge is working.

Citation list

Patent Literature

    PTL 1: WO2013/065528 corresponding to EP 2775307 A1 from which the pre-characterising part of claim 1 starts out.
    PTL 2: JP-A-2018-160808 Related art is disclosed in US 2019/0079107 A1.

Summary of Invention

Technical Problem

When handling the error, in order to prepare a reagent and a consumable necessary for eliminating the error, the person-in-charge may need to stop at another location such as a reagent storage or a consumable storage before heading to a location where the analyzer generating the error is present. In particular, in a case of a large-scale clinical laboratory, movement of a laboratory technician in charge of the handling of the error also leads to time loss.

However, the techniques described in PTLs 1 and 2 have not considered a case where there is a need to access a location other than the device generating the error in order to handle the error, and therefore time loss may occur.

An object of the invention is to provide an automatic analysis system that can, when an error occurs in an analyzer, processing device, or the like in a clinical laboratory, optimize assignment of a person-in-charge who handles the error and reduce a time period required for handling the error, even if there is a need to access to a position other than the device generating the error in order to handle the error.

Solution to Problem

The above problem is solved by an automatic analysis system and an alarm handling method as set forth in the appended claims.

Advantageous Effect

A time period required for dealing with a case where there is a need to access to another position prior to a device generating an alarm can be reduced.

Other problems and novel characteristics will become apparent from a description of the present specification and the accompanying drawings.

Brief Description of Drawings

[FIG. 1] FIG. 1 shows an example of a clinical laboratory.
[FIG. 2] FIG. 2 is a block diagram showing a schematic configuration of an automatic analysis system.
[FIG. 3] FIG. 3 shows an example of an alarm information management table.
[FIG. 4] FIG. 4 shows an example of a person-in-charge information management table.
[FIG. 5] FIG. 5 shows an example of a person-in-charge schedule management table.
[FIG. 6] FIG. 6 shows an example of an alarm handling stop position management table.
[FIG. 7] FIG. 7 shows an example of a clinical laboratory map.
[FIG. 8] FIG. 8 shows an example of an alarm notification screen.
[FIG. 9] FIG. 9 shows an example of an alarm handling answer screen.
[FIG. 10] FIG. 10 shows an example of a past comment confirmation screen.
[FIG. 11] FIG. 11 shows an example of alarm notification processing logic.
[FIG. 12] FIG. 12 shows an example of alarm notification order determination processing logic.
[FIG. 13] FIG. 13 shows an example of an alarm information management table.
[FIG. 14] FIG. 14 shows an example of a person-in-charge schedule management table.
[FIG. 15] FIG. 15 shows an example of an alarm information management table.
[FIG. 16] FIG. 16 shows an example of a person-in-charge schedule management table.
[FIG. 17] FIG. 17 shows an example of a person-in-charge schedule management table.
[FIG. 18] FIG. 18 shows an example of an extended alarm information management table.
[FIG. 19] FIG. 19 shows an example of a person-in-charge information management table.
[FIG. 20] FIG. 20 shows an example of an extended alarm handling stop position management table.
[FIG. 21] FIG. 21 shows an example of a registration screen of an alarm that can be handled.
[FIG. 22] FIG. 22 shows an example of a registration information management table of the alarm that can be handled.
[FIG. 23] FIG. 23 shows an example of registration processing logic of the alarm that can be handled.
[FIG. 24] FIG. 24 shows an example of an all alarm confirmation screen.

Description of Embodiments

An automatic analysis system of this embodiment has a function of, when a device in a clinical laboratory generates an alarm, deciding a notification order of notifying an optimum laboratory technician of the alarm in consideration of information on laboratory technicians in the clinical laboratory, a schedule of the laboratory technicians, positions of the laboratory technicians, a position of the device generating the alarm, a position of a location to stop to handle the alarm, and the like, and notifying the laboratory technician who is determined as being optimal of the alarm.

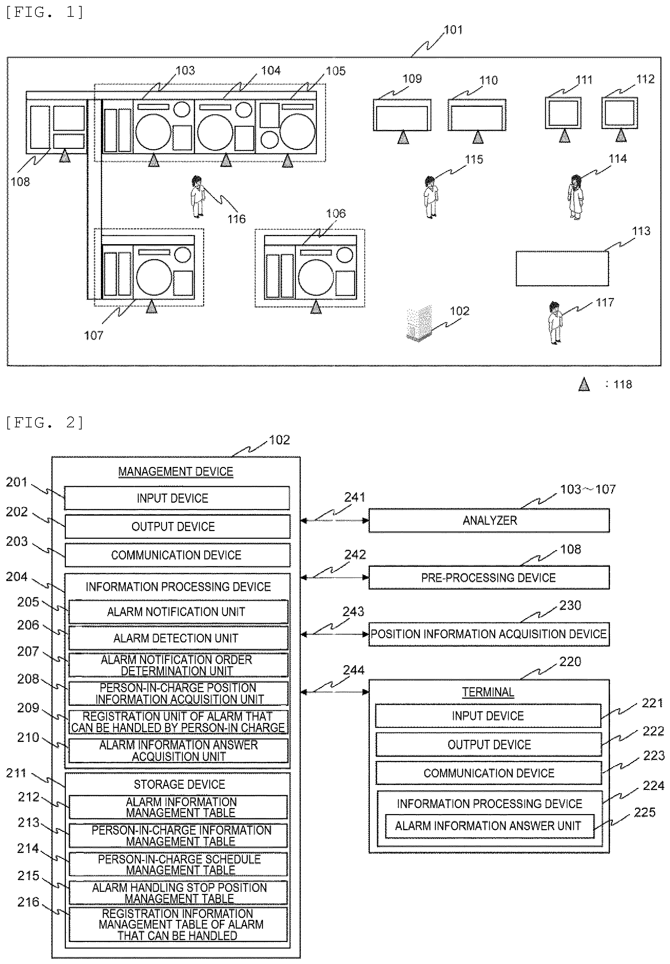

FIG. 1 shows an example of a clinical laboratory. A clinical laboratory 101, which is an area where clinical tests are performed, includes various devices, storages, a work table, and the like. The clinical laboratory 101 of FIG. 1 is provided with analyzers 103 to 107, a pre-processing device 108, reagent storages 109 and 110, consumable storages 111 and 112, and a work table 113. The analyzers are devices that perform the clinical tests, and may include both an analyzer that operates independently and an analyzer that operates in combination with a plurality of analyzers. In the example of FIG. 1, the analyzers 106 and 107 operate independently, and the analyzers 103 to 105 operate in combination. The pre-processing device 108 is a device that automatically performs pre-processing for the clinical tests, and is connected to the analyzers 103 to 105 and 107 by a transport mechanism. A pre-processed sample is transported to the analyzers by the transport mechanism. The reagent storages 109 and 110 store reagents necessary for performing analysis with the analyzers. The consumable storages 111 and 112 store consumables used by the analyzers and the pre-processing device. The work table 113 provides a space for the laboratory technicians to open a sample or the like to be transported into the clinical laboratory 101 and to perform other works. Each of the analyzers 103 to 105 and the pre-processing device 108 may be referred to as an analyzer in a broad sense.

In the clinical laboratory 101, a plurality of laboratory technicians 114 to 117 are working. The laboratory technicians are persons-in-charge who perform preparation for the clinical tests and operations on the analyzers and the pre-processing device, and each laboratory technician holds one or more terminals. The analyzers 103 to 107, the pre-processing device 108, the reagent storages 109 and 110, and the consumable storages 111 and 112 each have a front side, and the laboratory technicians move to the front side of these devices when performing works for the clinical tests or works for handling alarms from the devices. A front position of each device and storage, which is a work position of the laboratory technicians, is indicated by a triangle mark 118.

The clinical laboratory 101 is provided with a management device 102 that is connected to the devices and storages arranged in the clinical laboratory 101 and the terminals held by the laboratory technicians via networks, and manages clinical test operations in the clinical laboratory 101.

FIG. 2 is a block diagram showing a schematic configuration of an automatic analysis system that manages the clinical test operations in the clinical laboratory 101. A terminal 220 is a terminal held by each laboratory technician working in the clinical laboratory 101. In the automatic analysis system, the analyzers 103 to 107, the pre-processing device 108, the terminal 220 carried by the laboratory technician, and a position information acquisition device 230 that acquires position information of the laboratory technician in the clinical laboratory 101 are communicably connected to the management device 102 by networks 241 to 244. The position information acquisition device 230 is a device that acquires a site of the laboratory technician. A method for acquiring the position information is not limited. For example, the position information may be acquired using the terminal carried by the laboratory technician, or the position information of the laboratory technician may be acquired by image analysis of a surveillance camera image. For example, Global Positioning System (GPS), Beacon, Ultra Wide Band (UWB), Wi-Fi positioning, sonic positioning, dead reckoning, Indoor Messaging System (IMES), or a combination thereof can be used.

The automatic analysis system has a configuration depending on a configuration of the clinical laboratory managed by the management device 102, and thus does not necessarily include all the devices illustrated in FIG. 2, and may include devices not illustrated. For example, the management device 102 may be connected to an information system such as a clinical laboratory information system (LIS).

The management device 102 includes an input device 201, an output device 202, a communication device 203, an information processing device 204, and a storage device 211. The input device 201 is a device that receives instructions and information from an administrator or the laboratory technician, such as a keyboard, a mouse, and a screen touch panel of the output device 202. The output device 202 includes a monitor that displays input information or other result information, or a printer. The communication device 203 is a communication device by which the management device 102 communicates with other devices and terminals. For example, the communication device 203 is a communication device that connects to a wired/wireless local area network, a wired/wireless global area network, a mobile phone network, or the like. The information processing device 204 is a computer that executes various applications of the management device 102. The storage device 211 is a storage device that stores programs executed by the information processing device 204 and data, and can use, for example, a non-volatile memory such as an EEPROM or a flash memory, an HDD, or an SSD.

The management device 102 realizes functions thereof in cooperation with other hardware by the information processing device 204 executing the programs stored in the storage device 211. Each program executed by the information processing device 204, a function thereof, or a device for realizing the function may be referred to as a "function", a "unit", a "module", or the like. The management device 102 can be realized by, for example, a personal computer (PC) or a server.

Functions executed by the information processing device 204 include an alarm notification unit 205, an alarm detection unit 206, an alarm notification order determination unit 207, a person-in-charge position information acquisition unit 208, a registration unit of alarm that can be handled by person-in-charge 209, and an alarm information answer acquisition unit 210. The storage device 211 stores the programs for the information processing device 204 to execute these functions, and data including an alarm information management table 212, a person-in-charge information management table 213, a person-in-charge schedule management table 214, and alarm handling stop position management table 215, and a registration information management table of alarm that can be handled 216. Details of these will be described later. The above describes the function of notifying the laboratory technician of the alarm generated in the device in the clinical laboratory, which will be described as this embodiment, and the function that can be executed by the management device 102 is not limited thereto.

The laboratory technician receives a notification of the alarm generated in the device in the clinical laboratory via the terminal 220. Specifically, the laboratory technician can use the terminal 220 to view the alarm notified from the management device 102, handle the alarm, and give an answer about completion of the alarm handling. The input device 221, the output device 222, the communication device 223, and the information processing device 224 of the terminal 220 have the same functions as those of the management device 102, respectively. The alarm information answer unit 225 is a function executed by the information processing device 224 in this embodiment. The terminal 220 is preferably a mobile terminal or a wearable terminal carried by the laboratory technician, for example, but a notebook PC or desktop PC may also be used. Details of the alarm information answer unit 225 will be described later.

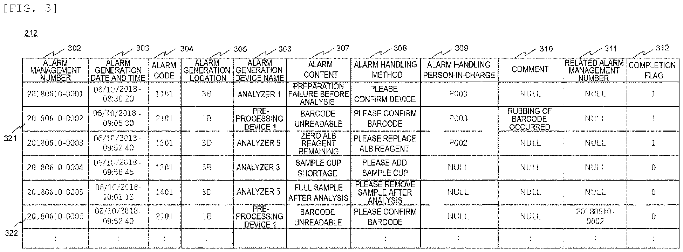

FIG. 3 shows an example of the alarm information management table 212. The alarm information management table 212 manages the alarm generated in the analyzers 103 to 107, the pre-processing device 108, or the management device 102. When the alarm detection unit 206 detects generation of the alarm in the management device 102 or receives a notification of the generation of the alarms from the other devices via the networks 241 and 242, information of the generated alarm is registered in the alarm information management table 212.

Column 302 is alarm management number and shows an identification number of the alarm generated in the clinical laboratory 101. Column 303 is alarm generation date and time and shows a date and time when the alarm generated in the clinical laboratory 101. Column 304 is alarm code and shows an identification number of a type of the alarm. Column 305 is alarm generation location and shows an identification number indicating a generation location of the alarm generated in the clinical laboratory 101. Column 306 is alarm generation device name, and shows a name of any of the analyzers, pre-processing device, and management device that generates the alarm in the clinical laboratory 101 in this case. Column 307 is alarm content, and shows a content of the alarm generated in the clinical laboratory 101. Column 308 is alarm handling method and shows a method for handling the generated alarm. The above pieces of information are registered in the alarm information management table 212 when the generation of the alarm is detected or the notification is received.

Column 309 is alarm handling person-in-charge and shows a person-in-charge who handles the alarm. NULL is registered as an initial value. Column 310 is comment and shows a comment of the alarm handling person-in-charge. NULL is registered as an initial value. Column 311 is related alarm management number, and registers an alarm management number of a related alarm. NULL is registered when there are no related alarms, such as a newly generated alarm. Column 312 is completion flag, and registers 1 indicating that handling is completed when the alarm handling person-in-charge completes or gives up the handling of the alarm. On the other hand, 0 indicating that the handling is not completed is registered as an initial value.

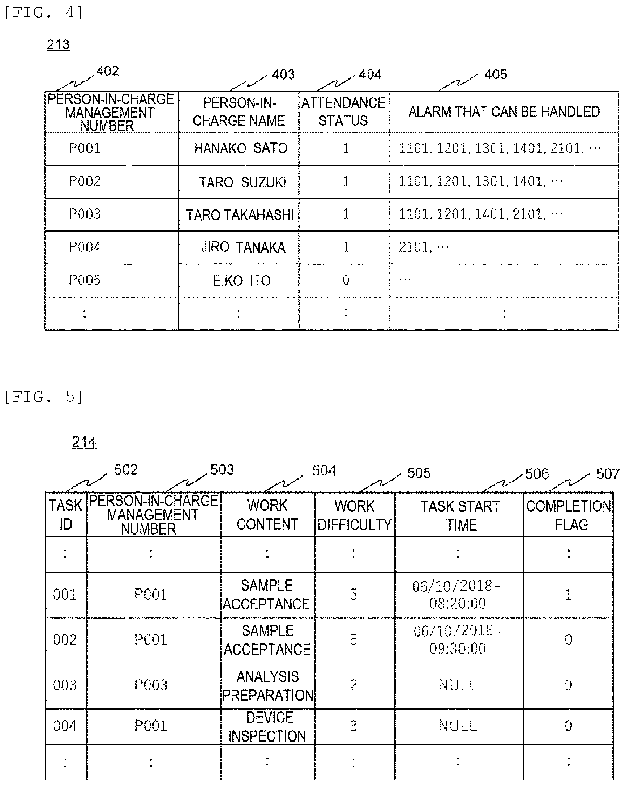

FIG. 4 shows an example of the person-in-charge information management table 213. The person-in-charge information management table 213 manages a name, an attendance status, and alarm codes of alarms that can be handled of each laboratory technician working in the clinical laboratory 101, and can identify a laboratory technician who can handle the alarm generated in the clinical laboratory 101.

Column 402 is person-in-charge management number and shows an identification number of the laboratory technician working in the clinical laboratory 101. Column 403 is person-in-charge name and shows the name of the laboratory technician working in the clinical laboratory 101. Column 404 is attendance status, and identifies whether the laboratory technician working in the clinical laboratory 101 is in attendance is identified. In this example, 1 is registered for an in-attendance status, 0 is registered for an out-of-attendance status, and an initial value is 0. The management device 102 acquires attendance information of the laboratory technician by detecting login/logoff of the terminal when the laboratory technician is in attendance or other methods, and updates the attendance status in the column 404. Column 405 is alarm that can be handled, and lists the alarm codes of the alarms that can be handled by the laboratory technician.

FIG. 5 shows an example of the person-in-charge schedule management table 214. The person-in-charge schedule management table 214 manages tasks in the clinical laboratory 101 and difficulty of each task, and is used to acquire a task that each laboratory technician is engaged in, including the difficulty of the task, when the alarm is generated. Column 502 is task ID and shows an identification number of each task in the clinical laboratory 101. Column 503 is person-in-charge management number and shows an identification number of the laboratory technician who performs the task. Column 504 is work content, that is, a task content. Column 505 is work difficulty. In this example, regarding the work difficulty, a task that takes more time to complete a work has greater difficulty, and a task that can be completed in a shorter time has smaller difficulty. For example, the difficulty is evaluated by 5 degrees of 5 to 1, and a higher score means greater difficulty. Column 506 is task start time and registers a time when the laboratory technician started the task. NULL is registered as an initial value when a new task is registered in the person-in-charge schedule management table 214, but when the management device 102 detects the task start time by a notification from the terminal carried by the laboratory technician or other methods, the task start time in the column 506 is updated. Column 507 is completion flag and manages whether the laboratory technician completes the task. Here, 0 indicating that the task is not completed is registered as an initial value, but when the management device 102 detects that the task is completed by the notification from the terminal carried by the laboratory technician or other methods, 1 indicating that the task is completed is registered.

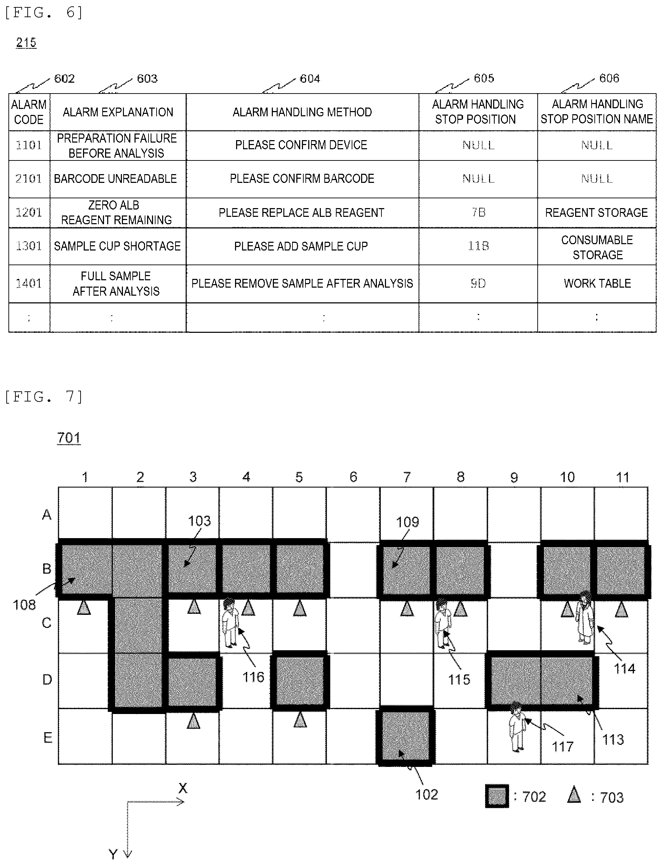

FIG. 6 shows an example of the alarm handling stop position management table 215. The alarm handling stop position management table 215 manages a location where the laboratory technician who handles the alarm stops prior to the device generating the alarm in order to handle the alarm. Column 602 is alarm code and shows an identification number of the alarm. Column 603 is alarm explanation and shows the content of the alarm. Column 604 is alarm handling method and shows a method for handling the alarm. Column 605 is alarm handling stop position, and registers a stop location for handling the alarm. When there is such a stop location for handling the alarm, an identification number indicating a position in the clinical laboratory 101 is registered, and there are no such stop locations for handling the alarm, NULL is registered. Column 606 is alarm handling stop position name, and registers a name of a corresponding location when an identification number is registered in the alarm handling stop position in the column 605.

FIG. 7 is an example of a clinical laboratory map 701. Each position in the clinical laboratory 101 is specified by an identification number defined on the clinical laboratory map. The clinical laboratory map 701 is a map of the clinical laboratory 101. In this example, the clinical laboratory 101 is divided into blocks, and the positions in the clinical laboratory 101 are specified by the identification numbers consisting of X coordinates and Y coordinates of the blocks. The X coordinates on the clinical laboratory map 701 are defined as numerical values 1 to 11 from left to right, and the Y coordinates are defined as alphabets A to E from top to bottom. For example, a current position of the laboratory technician 117 has an X coordinate 9 and a Y coordinate E, and thus is represented as an identification number 9E.

On the clinical laboratory map 701, an area indicated by hatching blocks 702 is provided with the devices/storages/work table, and the like, and the laboratory technicians cannot move in such an area. Since the triangle marks 703 indicate the work positions of the laboratory technicians, end points when the laboratory technicians move to the devices and the like to work are blocks marked with the triangle marks 703. For example, a route for the laboratory technician 116 to move to the pre-processing device 108 is 4C, 4D, 4E, 3E, 2E, 1E, 1D, and 1C, and a distance at that time is 7 because movement by 7 blocks is required. On the other hand, an end point of the work table which is not designated by the triangle mark 703 (work position) is set as a position of the corresponding hatching block. Further, when a plurality of hatching blocks are connected to each other, one of the hatching blocks is designated as an end point. For example, a block whose X coordinate value is smallest and Y coordinate alphabet is closest to A may be designated. Blocks where the management device 102 and the work table 113 are arranged correspond to blocks where no work positions are designated.

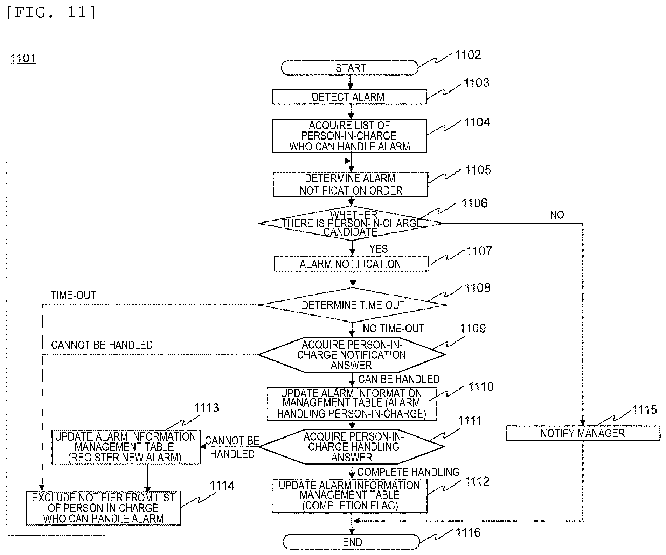

Hereinafter, alarm notification processing logic 1101 shown in FIG. 11 will be described. In step 1103 of detecting an alarm, the alarm detection unit 206 detects generation of an alarm or receives a notification of the generation of the alarm, and registers information on the generated alarm in the alarm information management table 212 (columns 302 to 308). The alarm notification order determination unit 207 executes the following steps when triggered by this registration.

In step 1104 of acquiring a list of person-in-charge who can handle the alarm, a list of laboratory technicians who can handle the alarm newly registered in the alarm information management table 212 is acquired. The list of person-in-charge who can handle the alarm is acquired from the person-in-charge information management table 213 by searching the alarm that can be handled (column 405) among the information on the persons-in-charge whose attendance status (column 404) is 1 by using the alarm code (column 304) in the alarm information management table 212 as a key.

In step 1105 of determining an alarm notification order, a notification priority is calculated for each laboratory technician in the list of person-in-charge who can handle the alarm created in step 1104. Details thereof will be described below.

In step 1106 of determining whether there is a person-in-charge candidate, whether there is a laboratory technician as a person-in-charge candidate is checked for the list of person-in-charge who can handle the alarm which is added with the notification priorities, and then the process is branched depending on results. When there are no person-in-charge candidates ("NO"), the alarm notification order determination unit 207 causes the alarm notification unit 205 to notify the administrator of the clinical laboratory 101 of the generation of the alarm (step 1115). At this time, it is desirable to attach a list of laboratory technicians who are in attendance.

Step 1115 of notifying the administrator is processing of notifying the administrator, who has an authority to make decisions about the operations of the clinical laboratory, that there is no laboratory technician who can handle the alarm. The administrator can forcibly decide an alarm handling person-in-charge, and also updates the alarm handling person-in-charge (column 309) in the alarm information management table 212. In addition, the administrator also can contact an external support when determining that the laboratory technicians in the clinical laboratory cannot handle the alarm. The administrator may be at any location either inside or outside the clinical laboratory.

On the other hand, when there is a person-in-charge candidate ("YES"), the alarm notification unit 205 is caused to notify a laboratory technician having a highest notification priority of the generation of the alarm (step 1107).

In step 1107 of notifying the alarm, the alarm notification unit 205 notifies a laboratory technician having a first priority in the list of person-in-charge who can handle the alarm. The alarm is notified to the terminal 220 held by the laboratory technician having the first priority via the network 244 by using the communication device 203.

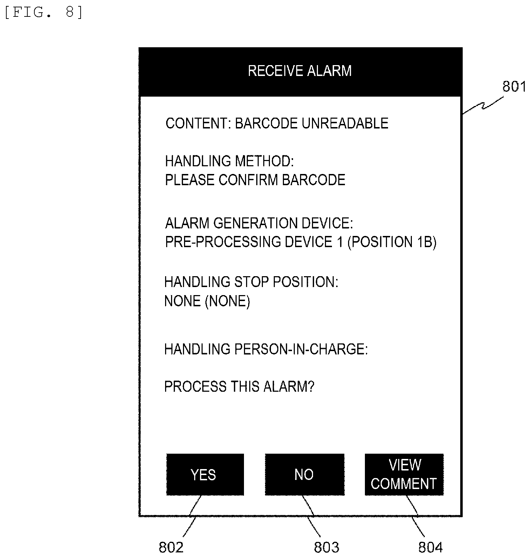

FIG. 8 is an alarm notification screen 801 displayed by the alarm information answer unit 225 on the terminal 220 held by the laboratory technician who is notified of the generation of the alarm. The alarm notification unit 205 notifies the terminal 220 of the laboratory technician of information related to the alarm based on the information registered in the alarm information management table 212 (see FIG. 3) and the alarm handling stop position management table 215 (see FIG. 6). The alarm notification screen 801 displays the information related to the alarm including the content, the handling method, the alarm generation device, the handling stop position, and the handling person-in-charge. At a stage of step 1107, the person-in-charge who handles the alarm is not decided, and thus is not displayed.

The laboratory technician notified of the generation of the alarm gives an answer of an intention to handle the alarm by the alarm notification screen 801. A button 802 is a "yes" button and notifies the management device 102 of an intention to be in charge. A button 803 is a "no" button and notifies the management device 102 of an intention not to be in charge. A button 804 is a "view comment" button, and is a button used to transition to a past comment confirmation screen 1001. Details will be described later. However, in this embodiment, when the alarm has started to be handled by another person-in-charge but is ended in a malfunction, a comment can be left about such a situation and the like. The laboratory technician can refer to this comment when determining whether or not to handle the alarm. Contents inputs by the buttons 802 to 804 are transmitted from the alarm information answer unit 225 of the terminal 220 to the alarm information answer acquisition unit 210 of the management device 102 via the network, and processing according to the input contents is performed.

Return to the description of the alarm notification processing logic 1101. In step 1108 of determining a time-out, a time-out of communication from the management device 102 to the terminal 220 is determined, and then the process is branched. When the communication is normally performed, the communication device 203 receives a notification that the communication is normally completed from the terminal 220. When the communication device 203 does not receive the notification that the communication is normally completed from the terminal 220 even after a period of time, it is regarded as a time-out. When a result of step 1108 of determining a time-out is "time-out", this notifier is excluded from the list of person-in-charge who can handle the alarm (step 1114). On the other hand, when the result of step 1108 of determining a time-out is "no time-out", processing of acquiring a person-in-charge notification answer is performed (step 1109).

In step 1109 of acquiring a person-in-charge notification answer, the alarm notification order determination unit 207 receives an answer of the intention to handle the alarm from the laboratory technician who is notified of the alarm from the alarm information answer acquisition unit 210, and then the process is branched. When the answer is that the alarm can be handled, processing of step 1110 of updating an alarm information management table (alarm handling person-in-charge) is performed. On the other hand, when the answer is that the alarm cannot be handled, this notifier is excluded from the list of person-in-charge who can handle the alarm (step 1114).

In step 1110 of updating the alarm information management table (alarm handling person-in-charge), the alarm notification order determination unit 207 registers the laboratory technicians who answer the intention to handle the notified alarm in the alarm handling person-in-charge (column 309) in the alarm information management table 212. Then, wait until contact from a handling person-in-charge is received.

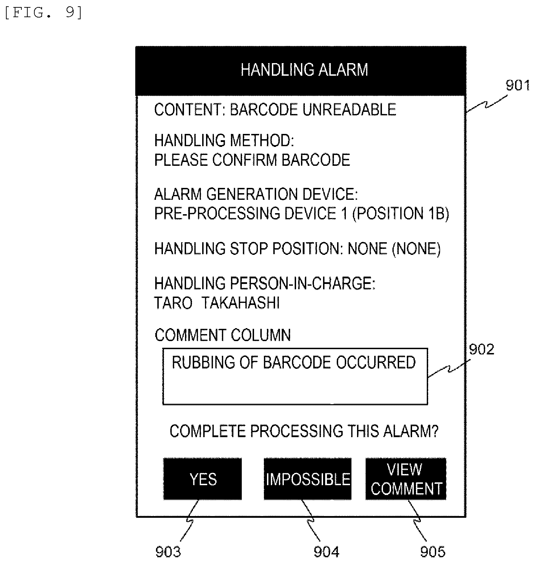

FIG. 9 shows an alarm handling answer screen 901 displayed by the alarm information answer unit 225 on the terminal 220 held by a laboratory technician who is in charge of the alarm. The laboratory technician gives an answer to the management device 102 that the handling of the alarm is completed through the alarm handling answer screen 901 when completing the handling of the alarm. A comment column 902 is a comment box in which the laboratory technician who is handling the alarm can leave a comment. The comment is input optionally, and can be input in either a case where the handling is completed or a case where the alarm cannot be handled. A button 903 is a "yes" button and notifies the management device 102 that the handling of the alarm is completed. A button 904 is an "impossible" button and notifies the management device 102 of an intention to give up the handling of the alarm (the alarm cannot be handled). A button 905 is a "view comment" button, and is a button used to transition to the past comment confirmation screen 1001. Contents input by the buttons 903 to 905 are transmitted from the alarm information answer unit 225 of the terminal 220 to the alarm information answer acquisition unit 210 of the management device 102 via the network, and processing according to the input contents is performed.

In step 1111 of acquiring a person-in-charge handling answer, the alarm notification order determination unit 207 receives an answer regarding the completion of the handling of the alarm from the laboratory technician who is the alarm handling person-in-charge from the alarm information answer acquisition unit 210, and then the process is branched. When the answer is that the handling is completed, processing of step 1112 of updating the alarm information management table (completion flag) is performed. On the other hand, when the answer is that the alarm cannot be handled (the laboratory technician gives up), processing of step 1113 of updating the alarm information management table (registering a new alarm) is performed.

In step 1112 of updating the alarm information management table (completion flag), the alarm notification order determination unit 207 updates the completion flag (column 312) in the alarm information management table 212 to 1 indicating that the handling is completed. When a comment is written in the comment column 902 of the alarm handling answer screen 901, the written comment is registered in the comment (column 310) in the alarm information management table 212, and the handling of the alarm is completed.

Step 1113 of updating the alarm information management table (registering a new alarm) is processing performed when the handling person-in-charge gives up to handle the alarm in step 1111 of acquiring a person-in-charge handling answer. The alarm notification order determination unit 207 registers the comment input into the comment column 902 on the alarm handling answer screen 901 in the comment (column 310) of the alarm information management table 212. Then, 1 indicating that the handling is completed is registered in the completion flag (column 312) of the alarm information management table 212. Then, the alarm that cannot be handled is again assigned a new alarm management number (column 302) as a new alarm, and information on the new alarm is registered in the alarm information management table 212. Registered contents of the information on the new alarm re-registered in the columns 303 to 308 of the alarm information management table 212 are the same as those of the alarm information on the alarm that cannot be handled (the laboratory technician gives up). In addition, an alarm management number of the alarm information of the alarm that cannot be handled is registered in the related alarm management number (column 311). Thereby, it is associated that the re-registered alarm information is the same as the information in the alarm that cannot be handled. After that, processing of step 1114 of excluding a notifier from the list of person-in-charge who can handle the alarm is performed.

Step 1114 of excluding the notifier is processing of excluding a currently notified laboratory technician from the list of person-in-charge who can handle the alarm. That is, a laboratory technician having a first notification priority is excluded from the list of person-in-charge who can handle the alarm in order of the notification priority. This step is executed when the alarm notification is time-out, when the notifier answers that the alarm cannot be handled, or when the handling person-in-charge answers that the alarm cannot be handled. The notifier is excluded from the list of person-in-charge who can handle the alarm, and then the process proceeds to step 1105 of determining an alarm notification order again.

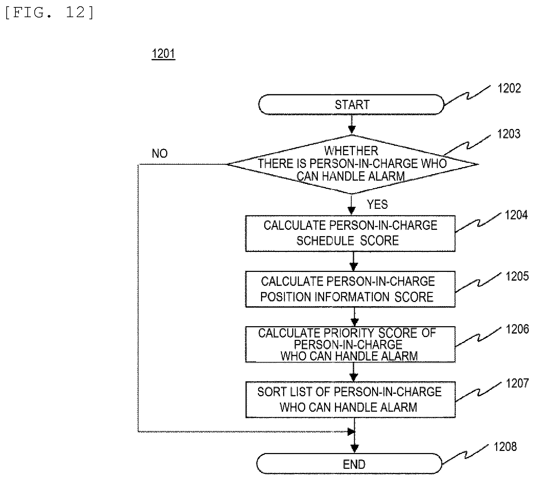

FIG. 12 shows alarm notification order determination processing logic 1201 executed by the alarm notification order determination unit 207. The alarm notification order determination processing logic 1201 is shown by a flowchart showing an example of step 1105 of determining an alarm notification order in the alarm notification processing logic 1101 (FIG. 11).

In step 1203 of determining whether there is a person-in-charge who can handle the alarm, it is checked whether there is a laboratory technician in the list of person-in-charge who can handle the alarm, and then the process is branched. When there is no person-in-charge who can handle the alarm ("NO"), the processing is ended. When there is a person-in-charge who can handle the alarm ("YES"), processing of step 1204 of calculating a person-in-charge schedule score is performed.

In step 1204 of calculating a person-in-charge schedule score, for each laboratory technician in the list of person-in-charge who can handle the alarm, a task currently being worked on and difficulty of the task are acquired from the person-in-charge schedule management table 214 (FIG. 5) by using the person-in-charge management number as a key, and a person-in-charge schedule score s is calculated. The task can be determined as currently being worked on when the task start time (column 506) is other than NULL and 0 indicating that the task is not completed is registered in the completion flag (column 507). The work difficulty is obtained from the column 505. A value of the work difficulty is used as it is as the person-in-charge schedule score s. Therefore, a task having a great work difficulty, that is, taking a long time to be completed has a high score. When there is no task currently being worked on, the score is set as 0 points.

In step 1205 of calculating a person-in-charge position information score, for each laboratory technician in the list of person-in-charge who can handle the alarm, a person-in-charge position information score p is calculated based on current position information of the laboratory technician. The position information of the laboratory technician is acquired by the person-in-charge position information acquisition unit 208 from the position information acquisition device 230 as the identification number represented by the clinical laboratory map 701. Then, the alarm handling stop position management table 215 (FIG. 6) is searched by using an alarm code of a currently generating alarm as a key, and an alarm handling stop position registered in the column 605 is acquired as a position information reference point. When the alarm handling stop position (column 605) is NULL, a location of a device generating the alarm (alarm generation location (column 305) in the alarm information management table 212 (FIG. 3)) is acquired as the position information reference point. Then, a shortest distance for the laboratory technician to reach the position information reference point is calculated based on the acquired position information reference point and the current position information of the laboratory technician. The number of blocks that the laboratory technician passes through from a block where the laboratory technician is currently located to a block where the position information reference point is located is set as the person-in-charge position information score p.

Step 1206 of calculating a priority score of a person-in-charge who can handle the alarm is processing in which for each laboratory technician in the list of person-in-charge who can handle the alarm, the person-in-charge schedule score s and the person-in-charge position information score p are input to calculate a notification priority score for deciding a notification priority of the alarm. For example, a notification priority score Pr is obtained by the following equation.

$$Pr = m_1 \cdot s + m_2 \cdot p \quad (Equation\ 1)$$

Here, $m_1$ is a weight for the person-in-charge schedule score s represented by a positive constant, and $m_2$ is a weight for the person-in-charge position information score p represented by a positive constant. In this case, a smaller notification priority score Pr indicates a higher priority, and a larger notification priority score Pr indicates a lower priority.

Step 1207 of sorting the list of person-in-charge who can handle the alarm is processing of sorting the list of person-in-charge who can handle the alarm in the order of the notification priority. When the notification priority score Pr is used as the notification priority of the persons-in-charge who can handle the alarm, a lower score indicates a higher priority, and a higher score indicates a lower priority. Therefore, the list of person-in-charge who can handle the alarm is sorted in an ascending order of the notification priority score Pr.

Hereinafter, application examples of the automatic analysis system of this embodiment will be described assuming some specific situations.

(Example of alarm notification when there is no location to stop for alarm handling)

An application example of the automatic analysis system of this embodiment will be described by taking a case of handling an alarm of preparation failure before analysis generated in the analyzer 103 as an example.

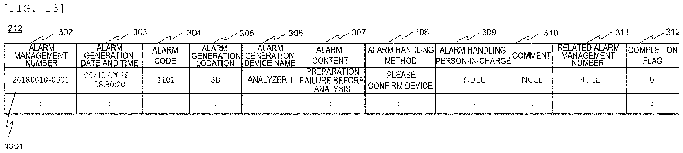

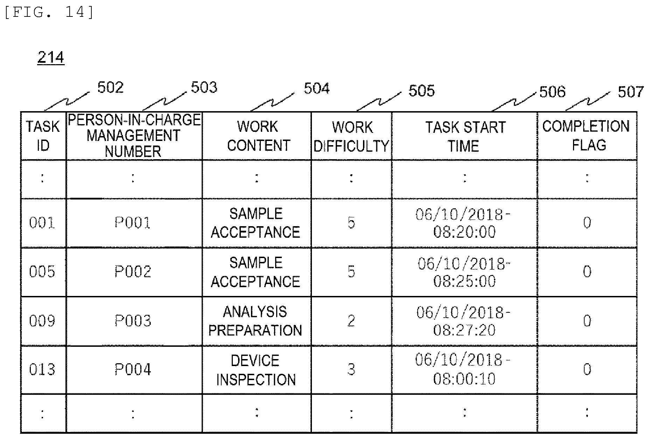

FIG. 13 shows alarm information 1301 in the alarm information management table 212, which indicates that the alarm of failure before analysis is generated in the analyzer 103. A person-in-charge schedule management table 214 at this time is shown in FIG. 14. The person-in-charge management numbers registered in the column 503 correspond to the laboratory technicians shown in FIG. 1, where P001 is the laboratory technician 114, P002 is the laboratory technician 115, P003 is the laboratory technician 116, and P004 is the laboratory technician 117. The same applies to the following examples.

The analyzer 103 starts an analysis operation in response to an instruction to start analysis from an operation screen of the analyzer 103 or the management device 102. At the start of the analysis operation, the analyzer 103 performs a reset operation. When an abnormality is detected in the reset operation, the analyzer 103 notifies the management device 102 of the alarm of preparation failure before analysis via the network 241. The management device 102 receives the alarm from the analyzer 103 and registers the alarm in the alarm information management table 212. A laboratory technician who is an alarm handling person-in-charge needs to move to the analyzer 103 in order to identify a cause of the failure of the reset operation, and handle the alarm.

The management device 102 uses an alarm code "1101" (column 304) registered in the alarm information 1301 as a key to acquire a list of persons-in-charge each having an alarm code matching this alarm code 1101 in the alarm that can be handled (column 405) in the person-in-charge information management table 213 (FIG. 4). In this example, the laboratory technicians having the person-in-charge management numbers P001, P002, and P003 can handle the alarm of preparation failure before analysis.

The management device 102 calculates the person-in-charge schedule scores s of the laboratory technicians who can handle the alarm of preparation failure before analysis. From the difficulty of each task currently being worked on, which is acquired from the person-in-charge schedule management table 214 (FIG. 14), the person-in-charge schedule scores s of the laboratory technicians having the person-in-charge management numbers P001, P002, and P003 are 5 points, 5 points, and 2 points, respectively.

Next, the person-in-charge position information scores p of the laboratory technicians who can handle the alarm of preparation failure before analysis are calculated. When the alarm handling stop position management table 215 (FIG. 6) is searched with the alarm code "1101", the alarm handling stop position is NULL, and therefore the position information reference point is 3C which is the front position of the analyzer 103 generating the alarm (see FIG. 7). According to the clinical laboratory map 701, the current positions of the laboratory technicians (114, 115, and 116) having the person-in-charge management numbers P001, P002, and P003 are 10C, 8C, and 4C, respectively. Therefore, the person-in-charge position information scores p of the laboratory technicians having the person-in-charge management numbers P001, P002, and P003 are 7 points, 5 points, and 1 point, respectively.

The notification priority score Pr is obtained by (Equation 1), where $m_1$ and $m_2$ are both 1, and the alarm notification priority scores Pr of the laboratory technicians having the person-in-charge management numbers P001, P002, and P003 are 12 points, 10 points, and 3 points, respectively. A lower notification priority score Pr leads to determination of a higher alarm notification priority, and thus the laboratory technician 116 having the person-in-charge management number P003 is notified of the alarm of preparation failure before analysis.

(Example of alarm notification when there is location to stop for alarm handling)

An application example of the automatic analysis system of this embodiment will be described by taking a case of handling an alarm of zero ALB reagent remaining generated in the analyzer 107 as an example.

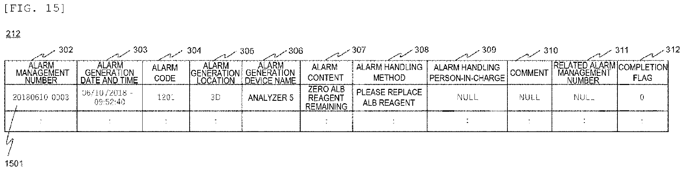

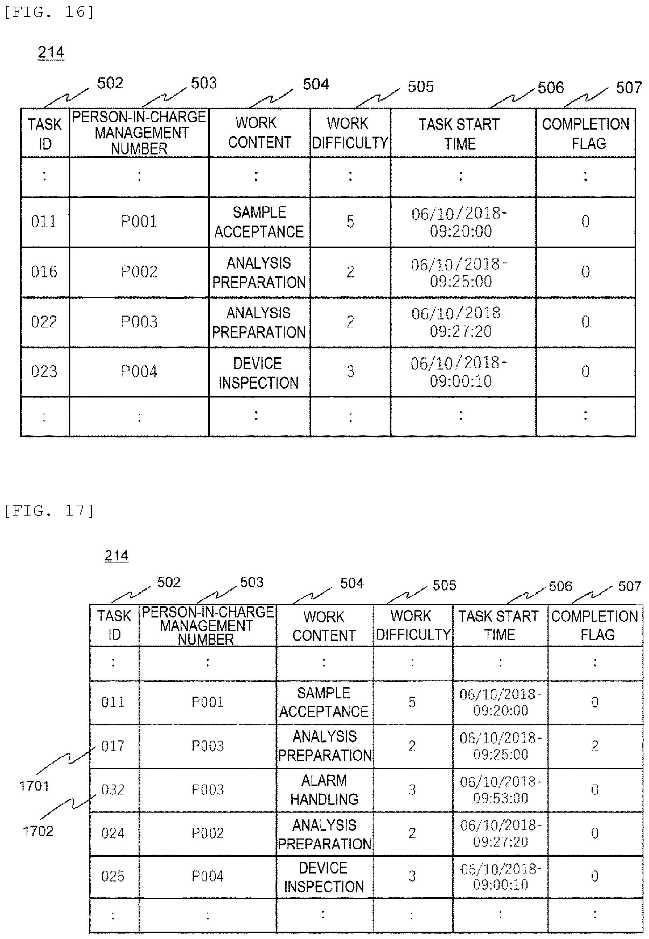

FIG. 15 shows alarm information 1501 in the alarm information management table 212, which indicates that the alarm of zero ALB reagent remaining is generated in the analyzer 107. A person-in-charge schedule management table 214 at this time is shown in FIG. 16.

The analyzer 107 starts an analysis operation in response to an instruction to start analysis from an operation screen of the analyzer 107 or the management device 102. An alarm of zero reagent remaining is generated when a remaining amount of a reagent required for analysis is low or zero. For example, when a remaining amount of an ALB reagent is zero, the alarm of zero ALB reagent remaining is generated, and the analyzer 107 notifies the management device 102 of the alarm of zero ALB reagent remaining via the network 241. The management device 102 receives the alarm from the analyzer 107 and registers the alarm in the alarm information management table 212. A laboratory technician who is an alarm handling person-in-charge needs to take out a new ALB reagent from the reagent storages 109 and 110 and put the new ALB reagent into the analyzer 107.

The management device 102 uses an alarm code "1201" (column 304) registered in the alarm information 1501 as a key to acquire a list of persons-in-charge each having an alarm code matching this alarm code 1201 in the alarms that can be handled (column 405) in the person-in-charge information management table 213 (FIG. 4). In this example, the laboratory technicians having the person-in-charge management numbers P001, P002, and P003 can handle the alarm of zero ALB reagent remaining.

The management device 102 calculates the person-in-charge schedule scores s of the laboratory technicians who can handle the alarm of zero ALB reagent remaining. From the difficulty of each task currently being worked on, which is acquired from the person-in-charge schedule management table 214 (FIG. 16), the person-in-charge schedule scores s of the laboratory technicians having the person-in-charge management numbers P001, P002, and P003 are 5 points, 2 points, and 2 points, respectively.

Next, the person-in-charge position information scores p of the laboratory technicians who can handle the alarm of zero ALB reagent remaining are calculated. When the alarm handling stop position management table 215 (FIG. 6) is searched with the alarm code "1201", the alarm handling stop position (column 605) is 7B, and therefore the position information reference point is 7C which is the front position of the reagent storage 109 (see FIG. 7). According to the clinical laboratory map 701, the current positions of the laboratory technicians (114, 115, and 116) having the person-in-charge management numbers P001, P002, and P003 are 10C, 8C, and 4C, respectively. Therefore, the person-in-charge position information scores p of the laboratory technicians having the person-in-charge management numbers P001, P002, and P003 are 3 points, 1 point, and 3 points, respectively.

The notification priority score Pr is obtained by (Equation 1), where $m_1$ and $m_2$ are both 1, and the alarm notification priority scores Pr of the laboratory technicians having the person-in-charge management numbers P001, P002, and P003 are 8 points, 3 points, and 5 points, respectively. A lower notification priority score Pr leads to determination of a higher alarm notification priority, and thus the laboratory technician 115 having the person-in-charge management number P002 is notified of the alarm of zero ALB reagent remaining.

(Example when alarm handling person-in-charge gives up handling of alarm)

An example when the laboratory technician 116 who is an alarm handling person-in-charge that handles an alarm of barcode unreadable generated in the pre-processing device 108 gives up handling of the alarm will be described.

FIG. 17 shows a person-in-charge schedule management table 214 when the laboratory technician 116 (person-in-charge management number P003) is in charge of the handling the alarm of barcode unreadable. Task ID 017 shows a task that is started before the laboratory technician 116 became the alarm handling person-in-charge. Since the laboratory technician 116 answers the management device 102 an intention of becoming the alarm handling person-in-charge, 2 indicating interruption is registered in the completion flag (column 507) in a record 1701, and a record 1702 showing an alarm handling task of task ID 032 is newly registered as being worked on.

The alarm of bar code unreadable is an alarm generated when a barcode attached to a test tube containing a sample cannot be read by the pre-processing device or the analyzer due to improper attachment position or dirt or rubbing on the barcode. When a barcode reading error occurs, the sample whose barcode is improper is not analyzed and is discharged to a sample container of the device. If the sample is not analyzed, diagnosis made by a doctor will be delayed, so a laboratory technician who is an alarm handling person-in-charge needs to move to the device generating the alarm, check the sample whose barcode cannot be read, and take measures such as re-sticking the barcode and reissuing the barcode.

At this time, assume that the laboratory technician 116 having the person-in-charge management number P003, who is the alarm handling person-in-charge of the alarm of barcode unreadable, cannot complete the handling of the alarm. For example, there is a case where the laboratory technician determines that the barcode needs to be reissued in order to handle the alarm, but does not know how to use a barcode issuing device. In this case, the laboratory technician 116 presses the button 904 on the alarm handling answer screen 901 of the terminal 220 to notify the management device 102 of giving up the handling of the alarm (see FIG. 9). At this time, the laboratory technician 116 can leave a comment in the comment column 902 for a person who will later become the alarm handling person-in-charge.

The management device 102 receives a notification from the laboratory technician having the person-in-charge management number P003 to give up the handling of the alarm, and updates the alarm information management table 212. The alarm information management table 212 in FIG. 3 is a table in a state where update of the alarm is completed. As shown in alarm information 321, a comment from the laboratory technician 116 is registered in the comment (column 310), and 1 indicating the completion of the handling of the alarm is registered in the completion flag (column 312). After that, the management device 102 newly adds the alarm of barcode unreadable to the alarm information management table 212. An alarm management number in the alarm information 321 is registered in the related alarm management number (column 311) of a newly added alarm information 322.

The management device 102 uses an alarm code "2101" (column 304) registered in the alarm information 322 as a key to acquire a list of persons-in-charge each having an alarm code matching this alarm code 2101 in the alarm that can be handled (column 405) in the person-in-charge information management table 213 (FIG. 4). In this example, the laboratory technicians having the person-in-charge management numbers P001, P003, and P004 can handle the alarm of barcode unreadable. Then, the related alarm management number (column 311) registered in the alarm information 322 is traced, and a past alarm handling person-in-charge is excluded. Therefore, the laboratory technicians having the person-in-charge management numbers P001 and P004 can handle the alarm of barcode unreadable.

In this case, the management device 102 calculates the person-in-charge schedule scores s, the person-in-charge position information scores p, and the person-in-charge priority scores Pr for the laboratory technicians having the person-in-charge management numbers P001 and P004, decides a laboratory technician to notify the alarm of barcode unreadable, and notifies the alarm.

The laboratory technician who is notified of the alarm views the alarm notification screen 801 through the terminal 220. The laboratory technician can optionally press the "view comment" button 804 to see the past comment confirmation screen 1001.

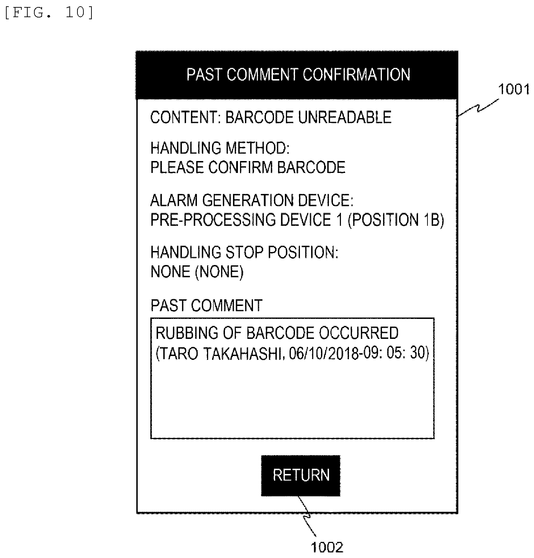

FIG. 10 shows an example of the past comment confirmation screen 1001. The laboratory technician can see the comment left by the past alarm handling person-in-charge (the laboratory technician having the person-in-charge management number P003 in this example) for the notified alarm. By pressing a "return" button 1002, the screen transitions to the alarm notification screen 801 again.

The screen can transition to the past comment confirmation screen 1001 from either the alarm notification screen 801 (FIG. 8) or the alarm handling answer screen 901 (FIG. 9) in FIG. 9. When the "return" button 1002 is pressed, the screen is transitioned to a transition source screen. In addition, the comment displayed on the past comment confirmation screen 1001 includes a comment registered in the comment (column 310) of the alarm information management table 212 and a comment registered in the comment (column 310) of the alarm information registered as a related alarm. Therefore, in this case, the comments registered in the comments (column 310) of the alarm information 321 and the alarm information 322 can be displayed on the past comment confirmation screen 1001 while the alarm having the alarm information 322 is being handled. More specifically, in the alarm information management table 212, all the registered comments (column 310) can be displayed on the past comment confirmation screen 1001 for related alarm information obtained by tracing the alarm information until the related alarm management number (column 311) becomes NULL by using the related alarm management number (column 311) in a current alarm information as a key.

(Example of alarm notification when notifying alarm to plural persons-in-charge)

An example when the analyzer 107 notifies an alarm of full sample after analysis will be described.

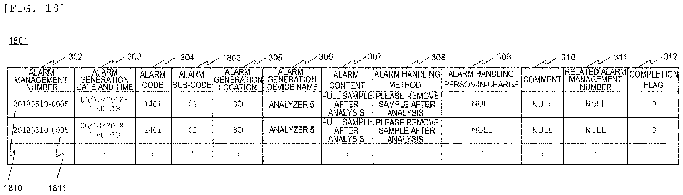

In the above examples, examples in which one laboratory technician is a handling person-in-charge of an alarm have been specifically described. However, it may be desirable for a plurality of laboratory technicians to handle an alarm in order to quickly handle the alarm. In this example, a case where alarm information includes information of the number of workers for alarm handling. FIG. 18 shows an extended alarm information management table 1801. The extended alarm information management table 1801 plays the same role as that of the alarm information management table 212, but is provided with an alarm sub-code (column 1804) that registers an identification number indicating that the number of workers for alarm handling is plural. In the extended alarm information management table 1801, when the alarm information shows that the number of worker for handling is one, NULL is registered. On the other hand, when the number of workers for alarm handling is plural, the plural pieces of alarm information are registered. For example, alarm information 1810 and alarm information 1811 have the same alarm management number (column 302) and relates to the same alarm, but this alarm is an alarm that requires two workers for alarm handling, and an alarm sub-code 01 is registered in the alarm information 1810 and an alarm sub-code 02 is registered in the alarm information 1811. The alarm information 1810 and the alarm information 1811 are treated as separate pieces of alarm information.

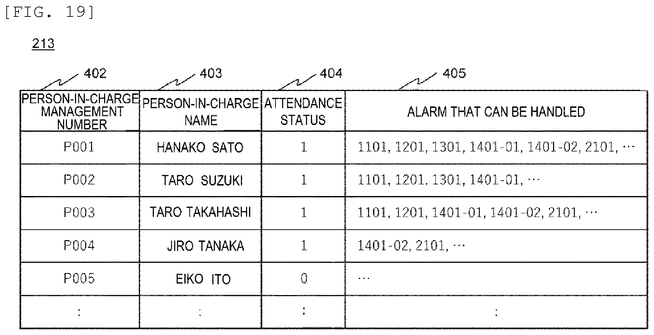

FIG. 19 shows a person-in-charge information management table 213 in this example. In this example, in the alarm that can be handled (column 405), a code obtained by combining an alarm code and an alarm sub-code of an alarm is registered for the alarm. For example, an alarm having an alarm code "1401" and an alarm sub-code "01" is registered as an alarm "1401-01" in the column 405. The reason why the alarm that can be handled is separately registered for each sub-alarm is that when the number of workers for alarm handling is plural, skills required of the handling persons-in-charge are not the same. For example, there may be a case where one person is a laboratory technician who is familiar with the alarm and one person is a work assistant to handle the alarm.

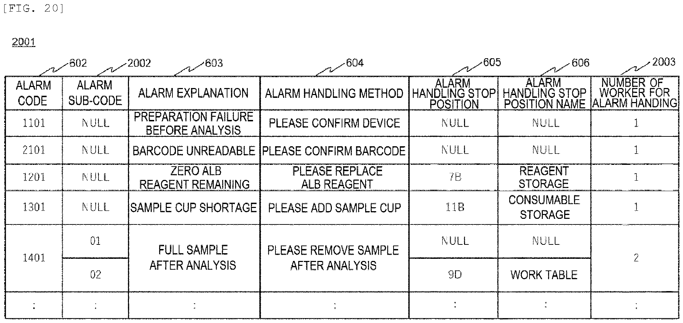

FIG. 20 shows an extended alarm handling stop position management table 2001. The extended alarm handling stop position management table 2001 plays the same role as that of the alarm handling stop position management table 215, but includes an alarm sub-code (column 2002) and the number of workers for alarm handling (column 2003) in which the number of workers necessary for handling the alarm is registered. When the number of workers for alarm handling **is 1,** NULL is stored in the alarm sub-code, and when the number of workers for alarm handling is plural, an alarm sub-code according to the number of the workers is stored. When the number of workers for alarm handling is plural, the handling stop position (column 605 and column 606) can be set for each alarm sub-code.

After analysis of a clinical test is completed, an analyzer temporarily stores the analyzed sample in the sample container in the device. Unfortunately, since the number of samples after analysis that can be temporarily stored is limited, the analyzer generates an alarm of full sample after analysis when the sample container is full. When the alarm of full sample after analysis is generated, a laboratory technician removes the sample after analysis from the analyzer and carries the sample after analysis to a work table. Therefore, it is necessary to clean up the work table in order to place the sample after analysis carried from the analyzer, and to clean up the sample after analysis after the sample after analysis arrives at the work table. Accordingly, in this example, in order to efficiently handle the alarm of full sample after analysis, two alarm handling persons-in-charge are needed including the laboratory technician who removes the sample after analysis from the analyzer and the laboratory technician who cleans up the work table and cleans up the sample after analysis.

When the alarm of full sample after analysis is generated in the analyzer 107, the analyzer 107 notifies the management device 102 of the alarm of full sample after analysis (alarm code "1401") via the network 241. The management device 102 receives the alarm from the analyzer 107 and registers the alarm in the extended alarm information management table 1801. In the extended alarm handling stop position management table 2001, the number of workers for alarm handling (column 2003) of the alarm code 1401 is 2, so two pieces of alarm information 1810 and 1811 of alarm sub-code 01 and alarm sub-code 02 are registered in the extended alarm information management table 1801. Since alarms having different alarm sub-codes are managed as separate alarms, it is necessary to notify the alarms to the two laboratory technicians who are the alarm handling persons-in-charge.

The management device 102 uses a code obtained by combining the alarm code "1401" (column 304) and the alarm sub-code "01" (column 1802) registered in the alarm information 1810 as a key to acquire a list of persons-in-charge each having an alarm code matching this code in the alarm that can be handled (column 405) in the person-in-charge information management table 213 (FIG. 19). In this example, the laboratory technicians having the person-in-charge management numbers P001, P002, and P003 can handle the alarm 1401-01. In the following, the laboratory technician to be notified of the alarm is decided and the alarm is notified by the same procedure as described above (example of alarm notification when there is no location to stop for alarm handling).

Then, a list of persons-in-charge having the alarm code matching alarm 1401-02 is acquired. In this example, the laboratory technicians having the person-in-charge management numbers P001, P003, and P004 can handle the alarm 1401-02. In the following, the laboratory technician to be notified of the alarm is decided and the alarm is notified by the same procedure as described above (example of alarm notification when there is a location to stop for alarm handling).

(Method for registering alarm that can be handled by laboratory technician)

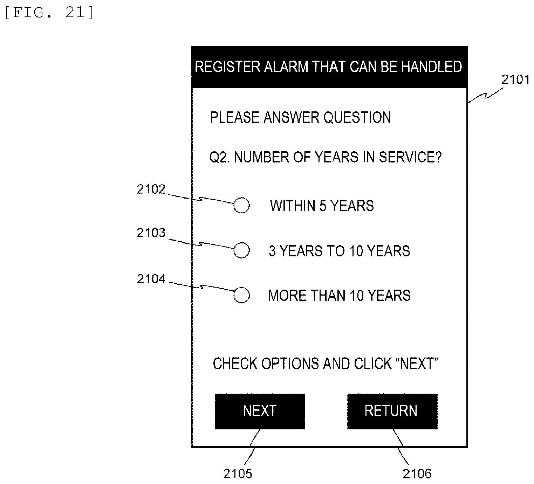

A method for registering an alarm that can be handled by a laboratory technician working in a clinical laboratory will be described with reference to the drawings of FIGS. 21 to 23. As a method for registering an alarm that can be handled by a laboratory technician, a method of this example can be used in addition to a method for registering an alarm for each laboratory technician by an administrator.

FIG. 21 shows a registration screen of alarm that can be handled 2101 displayed on the terminal 220 held by the laboratory technician. The laboratory technician working in the clinical laboratory 101 answers questions from the management device 102, and based on the answers, alarms that can be handled by the laboratory technician are registered in the alarm that can be handled (column 405) in the person-in-charge information management table 213. FIG. 21 shows an example of a question from the management device 102. The laboratory technician who answers the question answers the question by selecting one of radio buttons 2102 to 2104. Button 2105 is a "next" button, and the alarm information answer unit 225 of the terminal 220 notifies the management device 102 of a question No and an answer to this question. After that, the following question is displayed on the registration screen of alarm that can be handled 2101. The laboratory technician repeats answering as many times as the number of the questions from the management device 102. When the laboratory technician presses a "return" button 2106, a previous question is displayed, and the laboratory technician can modify the answer to the previous question.

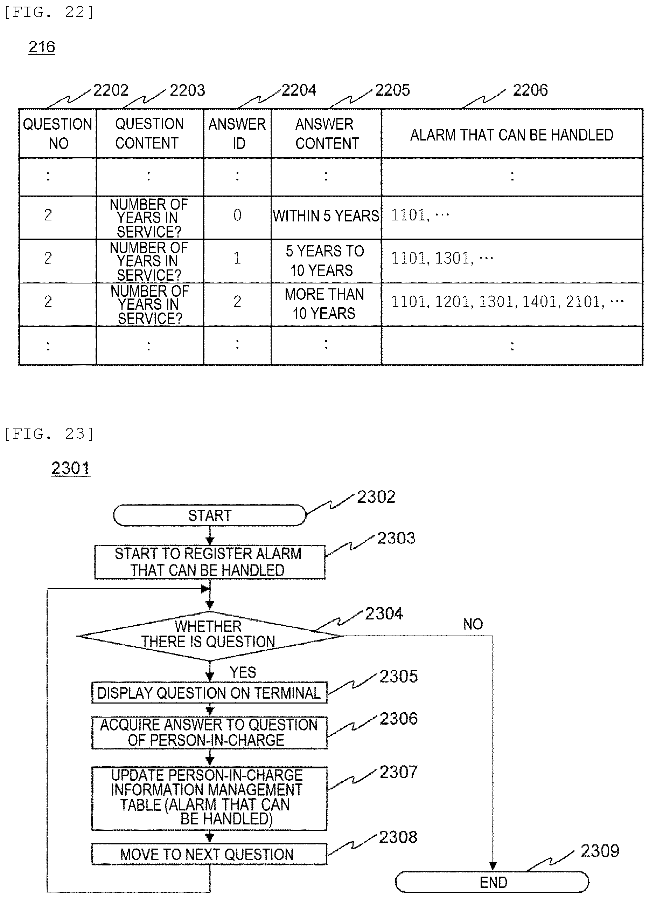

FIG. 22 shows the registration information management table of alarm that can be handled 216. The management device 102 updates the alarms that can be handled by the laboratory technician based on the registration information management table of alarm that can be handled 2201. Column 2202 is question No and shows an identification number of each question. Column 2203 is question content and shows a content of each question. Column 2204 is answer ID, and shows an identification number of an answer option for each question. Column 2205 is answer content, and shows an answer content of each question. Column 2206 is alarm that can be handled, and shows a list of the alarms registered in the alarm that can be handled (column 405) of the person-in-charge information management table 213.

FIG. 23 shows registration processing logic of alarm that can be handled 2301. The registration processing logic of alarm that can be handled 2301 is shown by a flowchart of processing executed by the registration unit of alarm that can be handled by person-in-charge 209 of the management device 102.

Step 2303 of starting to register an alarm that can be handled is processing in which the laboratory technician starts to register the alarm that can be handled through the terminal 220. The laboratory technician notifies the management device 102 of starting to register the alarm that can be handled from the terminal 220, and the registration unit of alarm that can be handled by person-in-charge 209 starts to send a question for registering the alarm that can be handled to the terminal 220.

In step 2304 of determining whether there is a question, whether there is a question to be sent is checked, and then the process is branched. When there is no question, the process is ended. When there is a question, the question is displayed on the terminal 220 (step 2305).

In step 2305 of displaying the question, the registration screen of alarm that can be handled 2101 is displayed on the terminal 220. The laboratory technician answers the question through the displayed registration screen of alarm that can be handled 2101.

In step 2306 of acquiring an answer to the question of the person-in-charge, the answer from the laboratory technician is acquired. The answer to the question from the terminal 220 includes the question No and the answer ID representing the answer.

In step 2307 of updating the person-in-charge information management table (alarm that can be handled), the registration unit of alarm that can be handled by person-in-charge 209 refers to the registration information management table of alarm that can be handled 216 based on the question No and the answer ID received from the terminal 220, and registers the alarms that can be handled in the alarm that can be handled (column 405) of the person-in-charge information management table 213. When the alarms are already registered in the alarm that can be handled (column 405), a logical sum of the already registered alarms to be handled and the alarms that can be handled acquired from the registration information management table of alarm that can be handled 216 is registered.

The process transitions to the next question (step 2308) and the above processing is repeated. When there is no next question, the process is ended (step 2309).

(Method for laboratory technician to confirm any alarm in laboratory)

By the automatic analysis system of this embodiment, each laboratory technician working in the clinical laboratory can confirm the alarms generating in the clinical laboratory 101 by his/her own terminal.

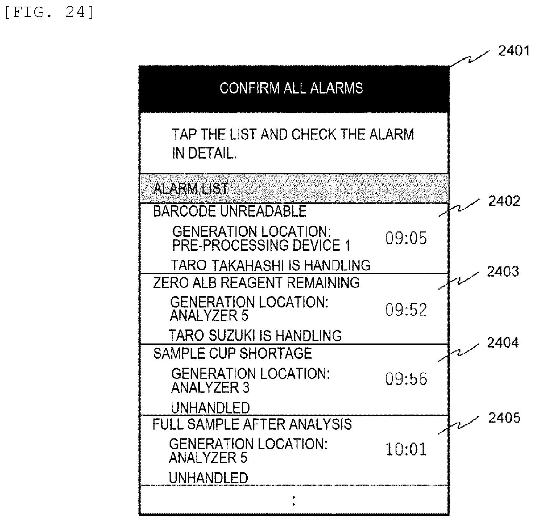

FIG. 24 shows an all alarm confirmation screen 2401 displayed on the terminal 220 held by each laboratory technician. The laboratory technician in the clinical laboratory 101 can confirm the status of the alarms generating in the laboratory through the all alarm confirmation screen 2401 by displaying the alarms in a list format. Elements 2402 to 2405 are elements of a list representing each alarm, and each list the alarm content, the alarm generation device name, an alarm handling status, and an alarm generation time. The names of the alarm handling persons-in-charge are displayed for the alarm handling status of the elements 2402 to 2405. When a new alarm is generated, an element representing the alarm is added to the all alarm confirmation screen 2401, and when the handling of the alarm is completed, the element of this alarm is deleted from the all alarm confirmation screen. Since the status of each alarm changes from moment to moment, the all alarm confirmation screen 2401 is updated at regular intervals while the all alarm confirmation screen 2401 is displayed for each laboratory technician.

The laboratory technician can also tap an element of an alarm for which the details are to be confirmed, so as to confirm the details. When the alarm tapped by the laboratory technician is an alarm for which the handling person-in-charge is decided, such as the elements 2402 and 2403, the screen transitions to the alarm handling answer screen 901 (FIG. 9) as a detailed confirmation screen of the alarm. However, when the laboratory technician who opened the alarm handling answer screen 901 from the all alarm confirmation screen 2401 is not the handling person-in-charge, the screen cannot be operated. In order to indicate that the screen cannot be operated, colors of the buttons or the like may be thinned or may not be displayed. When the alarm handling person-in-charge completes the handling of the alarm while the laboratory technician is checking the details of the alarm on the alarm handling answer screen 901, the management device 102 notifies the laboratory technician who is checking the details of the alarm that the handling of the alarm is completed, and causes the screen of the terminal 220 to transition to the all alarm confirmation screen 2401.

When the alarm tapped by the laboratory technician is an alarm for which the handling person-in-charge is not decided, such as the elements 2404 and 2405, the screen transitions to the alarm notification screen 801 (FIG. 8) as the detailed confirmation screen of the alarm. The laboratory technician can operate the alarm notification screen 801. When the "yes" button 802 is pressed, the management device 102 registers the laboratory technician as an alarm handling person-in-charge, and causes the screen of the terminal 220 to transition to the alarm handling answer screen 901. At this time, when the alarm notification processing logic 1101 shown in FIG. 11 is being executed in the management device 102, interrupt processing for ending a series of processing for notifying the alarm is performed, and the processing of the step 1111 of acquiring person-in-charge handling answer is started. When the processing of the step 1109 of acquiring person-in-charge notification answer is being processed, the laboratory technician who is notified of the alarm is notified that the handling person-in-charge is decided.

When the laboratory technician presses the "no" button 803, the screen of the terminal 220 transitions to the all alarm confirmation screen 2401. When the "view comment" button 804 is pressed, the screen of the terminal 220 transitions to the past comment confirmation screen 1001 (FIG. 10). When the alarm handling person-in-charge is decided while the laboratory technician is confirming the details of the alarm on the alarm notification screen 801, the management device 102 notifies the laboratory technician who is checking the details of the alarm that the alarm handling person-in-charge is decided, and causes the screen of the terminal 220 to transition to the all alarm confirmation screen 2401.

The invention has been described with reference to the embodiment, but the invention is not limited to the embodiment described above, and includes various modifications falling under the scope of the claims. The embodiment described above has been described for easy understanding of the invention, and is not necessarily limited to those including all the configurations described above.

Reference Sign List

101: clinical laboratory, 102: management device, 103, 104, 105, 106, 107: analyzer, 108: pre-processing device, 109, 110: reagent storage, 111, 112: consumable storage, 113: work table, 114, 115, 116, 117: laboratory technician, 118: front position, 201, 221: input device, 202, 222: output device, 203, 223: communication device, 204, 224: information processing device, 205: alarm notification unit, 206: alarm detection unit, 207: alarm notification order determination unit, 208: person-in-charge position information acquisition unit, 209: registration unit of alarm that can be handled by person-in charge, 210: alarm information answer acquisition unit, 211: storage device, 212: alarm information management table, 213: person-in-charge information management table, 214: person-in-charge schedule management table, 215: alarm handling stop position management table, 216: registration information management table of alarm that can be handled, 220: terminal, 225: alarm information answer unit, 230: position information acquisition device, 241, 242, 243, 244: network, 701: clinical laboratory map, 801: alarm notification screen, 901: alarm handling answer screen, 1001: past comment confirmation screen, 1101: alarm notification processing logic, 1201: alarm notification order determination processing logic, 1801: extended alarm information management table, 2001: extended alarm handling stop position management table, 2101: registration screen of alarm that can be handled, 2301: registration processing logic of alarm that can be handled, 2401: all alarm confirmation screen.

**Claims**

1.  An automatic analysis system comprising:

    an analyzer (103~107) disposed in a clinical laboratory (101) to analyze or pre-process a sample; and
    a management device (102) configured to manage the analyzer (103~107),
    wherein the management device (102) includes:

    a storage device (211) configured to store an alarm information management table (212) that registers information on an alarm whose generation is detected from the analyzer (103~107) or the management device (102), and
    an information processing device (204) configured to perform prioritization for notifying the alarm whose generation is detected on the laboratory technician (114~117) in the clinical laboratory (101),

    **characterized in that**
    the automatic analysis system comprises a plurality of said analyzers (103~107) managed by the management device (102),
    the storage device (211) is configured to further store a person-in-charge schedule management table (214) that registers a schedule of a laboratory technician (114~117) working in the clinical laboratory (101), and an alarm handling stop position management table (215) that registers information on a stop position (605) where the laboratory technician (114~117) who handles the alarm stops prior to a device that generates the alarm in order to handle the alarm generated from the plurality of analyzers (103~107) or the management device (102); and
    the information processing device (204) is configured to perform prioritization for notifying the alarm whose generation is detected on the laboratory technician (114~117) in the clinical laboratory (101), based on information on a task currently being performed by the laboratory technician (114~117) in the clinical laboratory (101), which is obtained from the person-in-charge schedule management table (214), and position information based on a distance according to a clinical laboratory map (701) between a current position of the laboratory technician (114~117) in the clinical laboratory (101) on the clinical laboratory map (701) and a position information reference point indicating a position (305, 605) on the clinical laboratory map (701) where the laboratory technician (114~117) who handles the alarm first stops, which is identified from the alarm information management table (212) and the alarm handling stop position management table (215).

2.  The automatic analysis system according to claim 1, wherein

    the storage device (211) stores a person-in-charge information management table (213) that registers the alarm that can be handled by the laboratory technician (114~117) working in the clinical laboratory (101), and the information processing device (204) narrows down the laboratory technician (114~117) as a candidate for notifying the alarm whose generation is detected based on the alarm information management table (212) and the person-in-charge information management table (213) among laboratory technicians (114~117) in the clinical laboratory (101).

3. The automatic analysis system according to claim 1, wherein

the laboratory technician (114~117) working in the clinical laboratory (101) holds a terminal (220), and the information processing device (204) notifies the terminal (220) of the laboratory technician (114~117) given a highest priority by the prioritization of the alarm whose generation is detected.

4. The automatic analysis system according to claim 3, wherein the information processing device (204) receives a notification of an intention to handle the alarm from the terminal (220) of the laboratory technician (114~117) notified of the alarm, and registers the laboratory technician (114~117) notified of the alarm as a person-in-charge who handles the alarm in the alarm information management table (212) stored in the storage device (211).

5. The automatic analysis system according to claim 4, wherein

the laboratory technician (114~117) as a person-in-charge who handles the alarm can input a comment on handling the alarm to the terminal (220), and the information processing device (204) receives input of the comment from the laboratory technician (114~117) as the person-in-charge who handles the alarm, and registers the comment in the alarm information management table (212) stored in the storage device (211).

6. The automatic analysis system according to claim 5, wherein the information processing device (204) receives a notification of an intention to give up handling the alarm from the terminal (220) of the laboratory technician (114~117) as the person-in-charge who handles the alarm, registers an alarm that the person-in-charge who handles the alarm gives up as a new alarm in the alarm information management table (212) stored in the storage device (211), and registers an alarm that the person-in-charge who handles the alarm gives up as a related alarm of the new alarm in the alarm information management table (212) stored in the storage device (211).

7. The automatic analysis system according to claim 2, wherein the information processing device (204) registers the information on the alarm whose generation is detected as n alarms in the alarm information management table (212) when the alarm whose generation is detected is an alarm that requires handling by n persons-in-charge, and performs the prioritization for notifying the alarm on the laboratory technician (114~117) in the clinical laboratory (101) for each of the n alarms.

8. The automatic analysis system according to claim 7, wherein in the person-in-charge information management table (213), when the alarm from the plurality of analyzers (103~107) or the management device (102) is an alarm that requires handling by n persons-in-charge, whether the alarm can be handled is registered for each of the n alarms registered in the alarm information management table (212).

9. An alarm handling method in a clinical laboratory (101) where a plurality of analyzers (103~107) configured to analyze or pre-process a sample are disposed and a plurality of laboratory technicians (114~117) work, comprising:

storing in advance a person-in-charge schedule management table (214) that registers a schedule of laboratory technicians (114~117) working in the clinical laboratory (101), and an alarm handling stop position management table (215) that registers information on a stop position (605) where a laboratory technician (114~117) who handles an alarm generated from the plurality of analyzers (103~107) stops prior to a device that generates the alarm in order to handle the alarm;
detecting (1103) generation of the alarm from the plurality of analyzers (103~107), and registering information of the alarm whose generation is detected in an alarm information management table (212); and
performing (1105) prioritization for notifying the alarm whose generation is detected on the laboratory technician (114~117) in the clinical laboratory (101), based on information on a task currently being performed by the laboratory technician (114~117) in the clinical laboratory (101), which is obtained from the person-in-charge schedule management table (214), and position information based on a distance according to a clinical laboratory map (701) between a current position of the laboratory technician (114~117) in the clinical laboratory (101) on the clinical laboratory map (701) and a position information reference point indicating a position (305, 605) on the clinical laboratory map (701) where the laboratory technician (114~117) who handles the alarm first stops, which is identified from the alarm information management table (212) and the alarm handling stop position management table (215).

10. The alarm handling method according to claim 9, wherein

a person-in-charge information management table (213) that registers the alarm that can be handled by the laboratory technician (114~117) working in the clinical laboratory (101) is stored in advance, and laboratory technicians (114~117) as candidates for notifying the alarm whose generation is detected are narrowed down from the alarm information management table (212) and the person-in-charge information management table (213).

11. The alarm handling method according to claim 9, wherein

the laboratory technician (114~117) working in the clinical laboratory (101) holds a terminal (220), and the alarm whose generation is detected is notified to the terminal (220) of the laboratory technician (114~117) given a highest priority by the prioritization.

12. The alarm handling method according to claim 11, wherein a notification of an intention to handle the alarm is received from the terminal (220) of the laboratory technician (114~117) notified of the alarm, and the laboratory technician (114~117) notified of the alarm is registered as a person-in-charge who handles the alarm in the alarm information management table (212).

13. The alarm handling method according to claim 12, wherein a notification of an intention to give up handling the alarm is received from the terminal (220) of the laboratory technician (114~117) as the person-in-charge who handles the alarm, an alarm that the person-in-charge who handles the alarm gives up is registered as a new alarm in the alarm information management table (212), and an alarm that the person-in-charge who handles the alarm gives up is registered as a related alarm of the new alarm in the alarm information management table (212).

14. The alarm handling method according to claim 10, wherein

generation of an alarm that requires n persons-in-charge who handle is detected from the plurality of analyzers (103~107), and information on the alarm whose generation is detected is registered as n alarms in the alarm information management table (212), and prioritization for notifying the alarm is performed on the laboratory technician (114~117) in the clinical laboratory (101) for each of the n alarms.

15. The alarm handling method according to claim 14, wherein in the person-in-charge information management table (213), when the alarm from the plurality of analyzers (103~107) is an alarm that requires handling by n persons-in-charge, whether the alarm can be handled is registered for each of the n alarms registered in the alarm information management table (212).

**Patentansprüche**

1. Automatisches Analysesystem, umfassend:

einen Analysator (103~107), der in einem Klinik-Labor (101) angeordnet ist, um eine Probe zu analysieren oder vorzuverarbeiten; und eine Verwaltungsvorrichtung (102), die dazu konfiguriert ist, den Analysator (103~107) zu verwalten, wobei die Verwaltungsvorrichtung (102) umfasst:

eine Speichervorrichtung (211), die dazu konfiguriert ist, eine Alarminformations-Verwaltungstabelle (212) zu speichern, die Informationen über einen Alarm registriert, dessen Erzeugung von dem Analysator (103~107) oder der Verwaltungsvorrichtung (102) erfasst wird, und eine Informationsverarbeitungsvorrichtung (204), die dazu konfiguriert ist, eine Priorisierung für die Benachrichtigung des Alarms, dessen Erzeugung erfasst wird, an den Labortechniker (114~117) in dem Klinik-Labor (101) durchzuführen,

**dadurch gekennzeichnet, dass**
das automatische Analysesystem mehrere der genannten Analysatoren (103~107) umfasst, die von der Verwaltungsvorrichtung (102) verwaltet werden,
die Speichervorrichtung (211) dazu konfiguriert ist, ferner eine Verantwortlichen-Zeitplan-Verwaltungstabelle (214) zu speichern, die einen Zeitplan eines Labortechnikers (114~117) registriert, der in dem Klinik-Labor (101) arbeitet, und eine Alarmbehandlungs-Stoppposition-Verwaltungstabelle (215), die Informationen über eine Stoppposition (605) registriert, an der der Labortechniker (114~117), der den Alarm behandelt, vor einer Vorrichtung, die den Alarm erzeugt, stoppt, um den Alarm zu behandeln, der von den mehreren Analysatoren (103~107) oder der Verwaltungsvorrichtung (102) erzeugt wird; und
die Informationsverarbeitungsvorrichtung (204) dazu konfiguriert ist, eine Priorisierung für die Benachrichtigung des Alarms, dessen Erzeugung erfasst wird, an den Labortechniker (114~117) in dem Klinik-Labor (101) durchzuführen, basierend auf Informationen über eine Aufgabe, die derzeit von dem Labortechniker (114~117) in dem Klinik-Labor (101) durchgeführt wird, die aus der Verantwortlichen-Zeitplan-Verwaltungstabelle (214) erhalten werden, und Positionsinformationen basierend auf einem Abstand gemäß einer Klinik-Laborkarte (701) zwischen einer aktuellen Position des Labortechnikers (114~117) in dem Klinik-Labor (101) auf der Klinik-Laborkarte (701) und einem Positionsinformations-Referenzpunkt, der eine Position (305, 605) auf der Klinik-Laborkarte (701) anzeigt, an der der Labortechniker (114~117), der den Alarm behandelt, zuerst stoppt, der aus der Alarminformations-Verwaltungstabelle (212) und der Alarmbehandlungs-Stoppposition-Verwaltungstabelle (215) identifiziert wird.

2. Automatisches Analysesystem nach Anspruch 1, wobei

die Speichervorrichtung (211) eine Verantwortlichen-Informations-Verwaltungstabelle (213) speichert, die den Alarm registriert, der von dem Labortechniker (114~117) behandelt werden kann, der in dem Klinik-Labor (101) arbeitet, und
die Informationsverarbeitungsvorrichtung (204) den Labortechniker (114~117) als Kandidaten für die Benachrichtigung des Alarms, dessen Erzeugung erfasst wird, basierend auf der Alarminformations-Verwaltungstabelle (212) und der Verantwortlichen-Informations-Verwaltungstabelle (213) unter den Labortechnikern (114~117) in dem Klinik-Labor (101) eingrenzt.

3. Automatisches Analysesystem nach Anspruch 1, wobei

der Labortechniker (114~117), der in dem Klinik-Labor (101) arbeitet, ein Terminal (220) trägt, und
die Informationsverarbeitungsvorrichtung (204) das Terminal (220) des Labortechnikers (114~117), dem durch die Priorisierung die höchste Priorität gegeben wird, über den Alarm benachrichtigt, dessen Erzeugung erfasst wird.

4. Automatisches Analysesystem nach Anspruch 3, wobei die Informationsverarbeitungsvorrichtung (204) eine Benachrichtigung über eine Absicht zur Behandlung des Alarms von dem Terminal (220) des Labortechnikers (114~117), der über den Alarm benachrichtigt wird, empfängt und den Labortechniker (114~117), der über den Alarm benachrichtigt wird, als Verantwortlichen, der den Alarm behandelt, in der Alarminformations-Verwaltungstabelle (212) registriert, die in der Speichervorrichtung (211) gespeichert ist.

5. Automatisches Analysesystem nach Anspruch 4, wobei

der Labortechniker (114~117) als Verantwortlicher, der den Alarm behandelt, einen Kommentar zur Behandlung des Alarms in das Terminal (220) eingeben kann, und
die Informationsverarbeitungsvorrichtung (204) die Eingabe des Kommentars von dem Labortechniker (114~117) als Verantwortlichen, der den Alarm behandelt, empfängt und den Kommentar in der Alarminformations-Verwaltungstabelle (212) registriert, die in der Speichervorrichtung (211) gespeichert ist.

6. Automatisches Analysesystem nach Anspruch 5, wobei die Informationsverarbeitungsvorrichtung (204) eine Benachrichtigung über eine Absicht zur Aufgabe der Behandlung des Alarms von dem Terminal (220) des Labortechnikers (114~117) als Verantwortlichen, der den Alarm behandelt, empfängt, einen Alarm, den der Verantwortliche, der den Alarm behandelt, aufgibt, als neuen Alarm in der Alarminformations-Verwaltungstabelle (212) registriert, die in der Speichervorrichtung (211) gespeichert ist, und einen Alarm, den der Verantwortliche, der den Alarm behandelt, aufgibt, als verwandten Alarm des neuen Alarms in der Alarminformations-Verwaltungstabelle (212) registriert, die in der Speichervorrichtung (211) gespeichert ist.

7. Automatisches Analysesystem nach Anspruch 2, wobei die Informationsverarbeitungsvorrichtung (204) die Informationen über den Alarm, dessen Erzeugung erfasst wird, als n Alarme in der Alarminformations-Verwaltungstabelle (212) registriert, wenn der Alarm, dessen Erzeugung erfasst wird, ein Alarm ist, der eine Behandlung durch n Verantwortliche erfordert, und die Priorisierung für die Benachrichtigung des Alarms an den Labortechniker (114~117) in dem Klinik-Labor (101) für jeden der n Alarme durchführt.

8. Automatisches Analysesystem nach Anspruch 7, wobei in der Verantwortlichen-Informations-Verwaltungstabelle (213), wenn der Alarm von den mehreren Analysatoren (103~107) oder der Verwaltungsvorrichtung (102) ein Alarm ist, der eine Behandlung durch n Verantwortliche erfordert, registriert wird, ob der Alarm für jeden der n Alarme behandelt werden kann, die in der Alarminformations-Verwaltungstabelle (212) registriert sind.

9. Alarmbehandlungsverfahren in einem Klinik-Labor (101), in dem mehrere Analysatoren (103~107), die dazu konfiguriert sind, eine Probe zu analysieren oder vorzuverarbeiten, angeordnet sind und mehrere Labortechniker (114~117) arbeiten, umfassend:

im-Voraus-Speichern einer Verantwortlichen-Zeitplan-Verwaltungstabelle (214), die einen Zeitplan von Labortechnikern (114~117) registriert, die in dem Klinik-Labor (101) arbeiten, und einer Alarmbehandlungs-Stoppposition-Verwaltungstabelle (215), die Informationen über eine Stoppposition (605) registriert, an der ein Labortechniker (114~117), der einen Alarm behandelt, der von den mehreren Analysatoren (103~107) erzeugt wird, vor einer Vorrichtung, die den Alarm erzeugt, stoppt, um den Alarm zu behandeln;
Erfassen (1103) der Erzeugung des Alarms von den mehreren Analysatoren (103~107) und Registrieren von Informationen des Alarms, dessen Erzeugung erfasst wird, in einer Alarminformations-Verwaltungstabelle (212); und
Durchführen (1105) einer Priorisierung für die Benachrichtigung des Alarms, dessen Erzeugung erfasst wird, an den Labortechniker (114~117) in dem Klinik-Labor (101), basierend auf Informationen über eine Aufgabe, die derzeit von dem Labortechniker (114~117) in dem Klinik-Labor (101) durchgeführt wird, die aus der Verantwortlichen-Zeitplan-Verwaltungstabelle (214) erhalten werden, und Positionsinformationen basierend auf einem Abstand gemäß einer Klinik-Laborkarte (701) zwischen einer aktuellen Position des Labortechnikers (114~117) in dem Klinik-Labor (101) auf der Klinik-Laborkarte (701) und einem Positionsinformations-Referenzpunkt, der eine Position (305, 605) auf der Klinik-Laborkarte (701) anzeigt, an der der Labortechniker (114~117), der den Alarm behandelt, zuerst stoppt, der aus der Alarminformations-Verwaltungstabelle (212) und der Alarmbehandlungs-Stoppposition-Verwaltungstabelle (215) identifiziert wird.

10. Alarmbehandlungsverfahren nach Anspruch 9, wobei

eine Verantwortlichen-Informations-Verwaltungstabelle (213), die den Alarm registriert, der von dem Labortechniker (114~117) behandelt werden kann, der in dem Klinik-Labor (101) arbeitet, im Voraus gespeichert wird, und
Labortechniker (114~117) als Kandidaten für die Benachrichtigung des Alarms, dessen Erzeugung erfasst wird, aus der Alarminformations-Verwaltungstabelle (212) und der Verantwortlichen-Informations-Verwaltungstabelle (213) eingegrenzt werden.

11. Alarmbehandlungsverfahren nach Anspruch 9, wobei

der Labortechniker (114~117), der in dem Klinik-Labor (101) arbeitet, ein Terminal (220) trägt, und
der Alarm, dessen Erzeugung erfasst wird, dem Terminal (220) des Labortechnikers (114~117) mitgeteilt wird, dem durch die Priorisierung die höchste Priorität gegeben wird.

12. Alarmbehandlungsverfahren nach Anspruch 11, wobei eine Benachrichtigung über eine Absicht zur Behandlung des Alarms von dem Terminal (220) des Labortechnikers (114~117), der über den Alarm benachrichtigt wird, empfangen wird, und der Labortechniker (114~117), der über den Alarm benachrichtigt wird, als Verantwortlicher, der den Alarm behandelt, in der Alarminformations-Verwaltungstabelle (212) registriert wird.

13. Alarmbehandlungsverfahren nach Anspruch 12, wobei eine Benachrichtigung über eine Absicht zur Aufgabe der Behandlung des Alarms von dem Terminal (220) des Labortechnikers (114~117) als Verantwortlichen, der den Alarm behandelt, empfangen wird, ein Alarm, den der Verantwortliche, der den Alarm behandelt, aufgibt, als neuer Alarm in der Alarminformations-Verwaltungstabelle (212) registriert wird, und ein Alarm, den der Verantwortliche, der den Alarm behandelt, aufgibt, als verwandter Alarm des neuen Alarms in der Alarminformations-Verwaltungstabelle (212) registriert wird.

14. Alarmbehandlungsverfahren nach Anspruch 10, wobei

die Erzeugung eines Alarms, der n behandelnde Verantwortliche erfordert, von den mehreren Analysatoren (103~107) erfasst wird, und Informationen über den Alarm, dessen Erzeugung erfasst wird, als n Alarme in der Alarminformations-Verwaltungstabelle (212) registriert werden, und
eine Priorisierung für die Benachrichtigung des Alarms an den Labortechniker (114~117) in dem Klinik-Labor (101) für jeden der n Alarme durchgeführt wird.

15. Alarmbehandlungsverfahren nach Anspruch 14, wobei in der Verantwortlichen-Informations-Verwaltungstabelle (213), wenn der Alarm von den mehreren Analysatoren (103~107) ein Alarm ist, der eine Behandlung durch n Verantwortliche erfordert, registriert wird, ob der Alarm für jeden der n Alarme behandelt werden kann, die in der Alarminformations-Verwaltungstabelle (212) registriert sind.

**Revendications**

1. Système d'analyse automatique comprenant :

un analyseur (103~107) disposé dans un laboratoire d'analyses (101) pour analyser ou prétraiter un échantillon ; et
un dispositif (102) de gestion configuré pour gérer l'analyseur (103~107),
dans lequel le dispositif (102) de gestion inclut :

un dispositif (211) de stockage configuré pour stocker une table (212) de gestion d'informations d'alarmes qui enregistre des informations sur un alarme dont la génération est détectée depuis l'analyseur (103~107) ou le dispositif (102) de gestion, et
un dispositif (204) de traitement d'informations configuré pour effectuer une priorisation pour notifier l'alarme dont la génération est détectée au technicien (114~117) de laboratoire dans le laboratoire d'analyses (101),

**caractérisé en ce que**
le système d'analyse automatique comprend une pluralité desdits analyseurs (103~107) gérés par le dispositif (102) de gestion,
le dispositif (211) de stockage est configuré pour stocker en outre une table (214) de gestion de planning de personnes responsables qui enregistre un planning d'un technicien (114~117) de laboratoire travaillant dans le laboratoire d'analyses (101), et une table (215) de gestion de positions d'arrêt de traitement d'alarme qui enregistre des informations sur une position (605) d'arrêt où le technicien (114~117) de laboratoire qui traite l'alarme s'arrête avant un dispositif qui génère l'alarme afin de traiter l'alarme générée depuis la pluralité d'analyseurs (103~107) ou le dispositif (102) de gestion ; et
le dispositif (204) de traitement d'informations est configuré pour effectuer une priorisation pour notifier l'alarme dont la génération est détectée au technicien (114~117) de laboratoire dans le laboratoire d'analyses (101), sur la base d'informations sur une tâche en cours d'exécution par le technicien (114~117) de laboratoire dans le laboratoire d'analyses (101), qui sont obtenues depuis la table (214) de gestion de planning de personnes responsables, et d'informations de position sur la base d'une distance conformément à une carte (701) de laboratoire d'analyses entre une position courante du technicien (114~117) de laboratoire dans le laboratoire d'analyses (101) sur la carte (701) de laboratoire d'analyses et un point de référence d'informations de position indiquant une position (305, 605) sur la carte (701) de laboratoire d'analyses où le technicien (114~117) de laboratoire qui traite l'alarme s'arrête en premier, qui est identifié à partir de la table (212) de gestion d'informations d'alarmes et de la table (215) de gestion de positions d'arrêt de traitement d'alarme.

2. Système d'analyse automatique selon la revendication 1, dans lequel

le dispositif (211) de stockage stocke une table (213) de gestion d'informations de personnes responsables qui enregistre l'alarme qui peut être traitée par le technicien (114~117) de laboratoire travaillant dans le laboratoire d'analyses (101), et

le dispositif (204) de traitement d'informations restreint le technicien (114~117) de laboratoire comme un candidat pour notifier l'alarme dont la génération est détectée sur la base de la table (212) de gestion d'informations d'alarmes et de la table (213) de gestion d'informations de personnes responsables parmi les techniciens (114~117) de laboratoire dans le laboratoire d'analyses (101).

3. Système d'analyse automatique selon la revendication 1, dans lequel

le technicien (114~117) de laboratoire travaillant dans le laboratoire d'analyses (101) dispose d'un terminal (220), et

le dispositif (204) de traitement d'informations notifie le terminal (220) du technicien (114~117) de laboratoire ayant reçu une priorité la plus élevée par la priorisation de l'alarme dont la génération est détectée.

4. Système d'analyse automatique selon la revendication 3, dans lequel le dispositif (204) de traitement d'informations reçoit une notification d'une intention de traiter l'alarme depuis le terminal (220) du technicien (114~117) de laboratoire notifié de l'alarme, et enregistre le technicien (114~117) de laboratoire notifié de l'alarme comme une personne responsable qui traite l'alarme dans la table (212) de gestion d'informations d'alarmes stockée dans le dispositif (211) de stockage.

5. Système d'analyse automatique selon la revendication 4, dans lequel

le technicien (114~117) de laboratoire comme une personne responsable qui traite l'alarme peut entrer un commentaire sur le traitement de l'alarme dans le terminal (220), et

le dispositif (204) de traitement d'informations reçoit l'entrée du commentaire du technicien (114~117) de laboratoire comme la personne responsable qui traite l'alarme, et enregistre le commentaire dans la table (212) de gestion d'informations d'alarmes stockée dans le dispositif (211) de stockage..

6. Système d'analyse automatique selon la revendication 5, dans lequel le dispositif (204) de traitement d'informations reçoit une notification d'une intention d'abandonner le traitement de l'alarme depuis le terminal (220) du technicien (114~117) de laboratoire comme la personne responsable qui traite l'alarme, enregistre une alarme que la personne responsable qui traite l'alarme abandonne comme une nouvelle alarme dans la table (212) de gestion d'informations d'alarmes stockée dans le dispositif (211) de stockage, et enregistre une alarme que la personne responsable qui traite l'alarme abandonne comme une alarme associée de la nouvelle alarme dans la table (212) de gestion d'informations d'alarmes stockée dans le dispositif (211) de stockage.

7. Système d'analyse automatique selon la revendication 2, dans lequel le dispositif (204) de traitement d'informations enregistre les informations sur l'alarme dont la génération est détectée comme n alarmes dans la table (212) de gestion d'informations d'alarmes lorsque l'alarme dont la génération est détectée est une alarme qui requiert un traitement par n personnes responsables, et effectue la priorisation pour notifier l'alarme au technicien (114~117) de laboratoire dans le laboratoire d'analyses (101) pour chacune des n alarmes.

8. Système d'analyse automatique selon la revendication 7, dans lequel, dans la table (213) de gestion d'informations de personnes responsables, lorsque l'alarme provenant de la pluralité d'analyseurs (103~107) ou du dispositif (102) de gestion est une alarme qui requiert un traitement par n personnes responsables, savoir si l'alarme peut être traitée est enregistré pour chacune des n alarmes enregistrées dans la table (212) de gestion d'informations d'alarmes.

9. Procédé de traitement d'alarmes dans un laboratoire d'analyses (101) où une pluralité d'analyseurs (103~107) configurés pour analyser ou prétraiter un échantillon sont disposés et une pluralité de techniciens (114~117) de laboratoire travaillent, comprenant :

le stockage au préalable d'une table (214) de gestion de planning de personnes responsables qui enregistre un planning de techniciens (114~117) de laboratoire travaillant dans le laboratoire d'analyses (101), et une table (215) de gestion de positions d'arrêt de traitement d'alarme qui enregistre des informations sur une position (605) d'arrêt où un technicien (114~117) de laboratoire qui traite une alarme générée depuis la pluralité d'analyseurs (103~107) s'arrête avant un dispositif qui génère l'alarme afin de traiter l'alarme ;

la détection (1103) de la génération de l'alarme depuis la pluralité d'analyseurs (103~107), et l'enregistrement d'informations de l'alarme dont la génération est détecté dans une table (212) de gestion d'informations d'alarmes ; et

l'exécution (1105) d'une priorisation pour notifier l'alarme dont la génération est détectée au technicien (114~117) de laboratoire dans le laboratoire d'analyses (101), sur la base d'informations sur une tâche en cours d'exécution par le technicien (114~117) de laboratoire dans le laboratoire d'analyses (101), qui sont obtenues depuis la table (214) de gestion de planning de personnes responsables, et d'informations de position sur la base d'une distance conformément à une carte (701) de laboratoire d'analyses entre une position courante du technicien (114~117) de laboratoire dans le laboratoire d'analyses (101) sur la carte (701) de laboratoire d'analyses et un point de référence d'informations de position indiquant une position (305, 605) sur la carte (701) de laboratoire d'analyses où le technicien (114~117) de laboratoire qui traite l'alarme s'arrête en premier, qui est identifié à partir de la table (212) de gestion d'informations d'alarmes et de la table (215) de gestion de positions d'arrêt de traitement d'alarme.

10. Procédé de traitement d'alarmes selon la revendication 9, dans lequel

une table (213) de gestion d'informations de personnes responsables qui enregistre l'alarme qui peut être traitée par le technicien (114~117) de laboratoire travaillant dans le laboratoire d'analyses (101) est stockée au préalable, et
des techniciens (114~117) de laboratoire comme candidats pour notifier l'alarme dont la génération est détectée sont restreints à partir de la table (212) de gestion d'informations d'alarmes et de la table (213) de gestion d'informations de personnes responsables.

11. Procédé de traitement d'alarmes selon la revendication 9, dans lequel

le technicien (114~117) de laboratoire travaillant dans le laboratoire d'analyses (101) dispose d'un terminal (220), et
l'alarme dont la génération est détectée est notifiée au terminal (220) du technicien (114~117) de laboratoire ayant reçu une priorité la plus élevée par la priorisation.

12. Procédé de traitement d'alarmes selon la revendication 11, dans lequel une notification d'une intention de traiter l'alarme est reçue depuis le terminal (220) du technicien (114~117) de laboratoire notifié de l'alarme, et le technicien (114~117) de laboratoire notifié de l'alarme est enregistré comme une personne responsable qui traite l'alarme dans la table (212) de gestion d'informations d'alarmes.

13. Procédé de traitement d'alarmes selon la revendication 12, dans lequel une notification d'une intention d'abandonner le traitement de l'alarme est reçue depuis le terminal (220) du technicien (114~117) de laboratoire comme la personne responsable qui traite l'alarme, une alarme que la personne responsable qui traite l'alarme abandonne est enregistrée comme une nouvelle alarme dans la table (212) de gestion d'informations d'alarmes, et une alarme que la personne responsable qui traite l'alarme abandonne est enregistrée comme une alarme associée de la nouvelle alarme dans la table (212) de gestion d'informations d'alarmes.

14. Procédé de traitement d'alarmes selon la revendication 10, dans lequel

la génération d'une alarme qui requiert n personnes responsables qui traitent est détectée depuis la pluralité d'analyseurs (103~107), et des informations sur l'alarme dont la génération est détectée sont enregistrées comme n alarmes dans la table (212) de gestion d'informations d'alarmes, et
une priorisation pour notifier l'alarme au technicien (114~117) de laboratoire dans le laboratoire d'analyses (101) est effectuée pour chacune des n alarmes.

15. Procédé de traitement d'alarmes selon la revendication 14, dans lequel, dans la table (213) de gestion d'informations de personnes responsables, lorsque l'alarme provenant de la pluralité d'analyseurs (103~107) est une alarme qui requiert un traitement par n personnes responsables, savoir si l'alarme peut être traitée est enregistré pour chacune des n alarmes enregistrées dans la table (212) de gestion d'informations d'alarmes.

[FIG. 1]

△ : 118

[FIG. 2]

[FIG. 3]

212

| ALARM MANAGEMENT NUMBER (302) | ALARM GENERATION DATE AND TIME (303) | ALARM CODE (304) | ALARM GENERATION LOCATION (305) | ALARM GENERATION DEVICE NAME (306) | ALARM CONTENT (307) | ALARM HANDLING METHOD (308) | ALARM HANDLING PERSON-IN-CHARGE (309) | COMMENT (310) | RELATED ALARM MANAGEMENT NUMBER (311) | COMPLETION FLAG (312) |
|---|---|---|---|---|---|---|---|---|---|---|
| 20180610-0001 | 06/10/2018 - 08:30:20 | 1101 | 3B | ANALYZER 1 | PREPARATION FAILURE BEFORE ANALYSIS | PLEASE CONFIRM DEVICE | P003 | NULL | NULL | 1 |
| 20180610-0002 | 06/10/2018 - 09:05:30 | 2101 | 1B | PRE-PROCESSING DEVICE 1 | BARCODE UNREADABLE | PLEASE CONFIRM BARCODE | P003 | RUBBING OF BARCODE OCCURRED | NULL | 1 |
| 20180610-0003 | 06/10/2018 - 09:52:40 | 1201 | 3D | ANALYZER 5 | ZERO ALB REAGENT REMAINING | PLEASE REPLACE ALB REAGENT | P002 | NULL | NULL | 1 |
| 20180610-0004 | 06/10/2018 - 09:55:45 | 1301 | 5B | ANALYZER 3 | SAMPLE CUP SHORTAGE | PLEASE ADD SAMPLE CUP | NULL | NULL | NULL | 0 |
| 20180610-0005 | 06/10/2018 - 10:01:13 | 1401 | 3D | ANALYZER 5 | FULL SAMPLE AFTER ANALYSIS | PLEASE REMOVE SAMPLE AFTER ANALYSIS | NULL | NULL | NULL | 0 |
| 20180610-0006 | 06/10/2018 - 09:52:40 | 2101 | 1B | PRE-PROCESSING DEVICE 1 | BARCODE UNREADABLE | PLEASE CONFIRM BARCODE | NULL | NULL | 20180610-0002 | 0 |
| : | : | : | : | : | : | : | : | : | : | : |

321

322

[FIG. 4]

<u>213</u>

| PERSON-IN-CHARGE MANAGEMENT NUMBER | PERSON-IN-CHARGE NAME | ATTENDANCE STATUS | ALARM THAT CAN BE HANDLED |
|---|---|---|---|
| P001 | HANAKO SATO | 1 | 1101, 1201, 1301, 1401, 2101, ⋯ |
| P002 | TARO SUZUKI | 1 | 1101, 1201, 1301, 1401, ⋯ |
| P003 | TARO TAKAHASHI | 1 | 1101, 1201, 1401, 2101, ⋯ |
| P004 | JIRO TANAKA | 1 | 2101, ⋯ |
| P005 | EIKO ITO | 0 | ⋯ |
| ⋮ | ⋮ | ⋮ | ⋮ |

[FIG. 5]

<u>214</u>

| TASK ID | PERSON-IN-CHARGE MANAGEMENT NUMBER | WORK CONTENT | WORK DIFFICULTY | TASK START TIME | COMPLETION FLAG |
|---|---|---|---|---|---|
| ⋮ | ⋮ | ⋮ | ⋮ | ⋮ | ⋮ |
| 001 | P001 | SAMPLE ACCEPTANCE | 5 | 06/10/2018-08:20:00 | 1 |
| 002 | P001 | SAMPLE ACCEPTANCE | 5 | 06/10/2018-09:30:00 | 0 |
| 003 | P003 | ANALYSIS PREPARATION | 2 | NULL | 0 |
| 004 | P001 | DEVICE INSPECTION | 3 | NULL | 0 |
| ⋮ | ⋮ | ⋮ | ⋮ | ⋮ | ⋮ |

[FIG. 6]

215

| ALARM CODE | ALARM EXPLANATION | ALARM HANDLING METHOD | ALARM HANDLING STOP POSITION | ALARM HANDLING STOP POSITION NAME |
|---|---|---|---|---|
| | 602 | 603 | 604 | 605 | 606 |
| 1101 | PREPARATION FAILURE BEFORE ANALYSIS | PLEASE CONFIRM DEVICE | NULL | NULL |
| 2101 | BARCODE UNREADABLE | PLEASE CONFIRM BARCODE | NULL | NULL |
| 1201 | ZERO ALB REAGENT REMAINING | PLEASE REPLACE ALB REAGENT | 7B | REAGENT STORAGE |
| 1301 | SAMPLE CUP SHORTAGE | PLEASE ADD SAMPLE CUP | 11B | CONSUMABLE STORAGE |
| 1401 | FULL SAMPLE AFTER ANALYSIS | PLEASE REMOVE SAMPLE AFTER ANALYSIS | 9D | WORK TABLE |
| ⋮ | ⋮ | ⋮ | ⋮ | ⋮ |

[FIG. 7]

701

■ : 702　△ : 703

[FIG. 8]

```
RECEIVE ALARM                        801


CONTENT: BARCODE UNREADABLE

HANDLING METHOD:
PLEASE CONFIRM BARCODE


ALARM GENERATION DEVICE:
PRE-PROCESSING DEVICE 1 (POSITION 1B)


HANDLING STOP POSITION:
NONE (NONE)


HANDLING PERSON-IN-CHARGE:

PROCESS THIS ALARM?



       YES         NO        VIEW
                           COMMENT


       802         803        804
```

[FIG. 9]

---

**HANDLING ALARM**

CONTENT: BARCODE UNREADABLE

HANDLING METHOD:
PLEASE CONFIRM BARCODE

ALARM GENERATION DEVICE:
PRE-PROCESSING DEVICE 1 (POSITION 1B)

HANDLING STOP POSITION: NONE (NONE)

HANDLING PERSON-IN-CHARGE:
TARO  TAKAHASHI

COMMENT COLUMN

| RUBBING OF BARCODE OCCURRED |

COMPLETE PROCESSING THIS ALARM?

| YES | IMPOSSIBLE | VIEW COMMENT |

901
902
903
904
905

[FIG. 10]

```
┌─────────────────────────────────────────┐
│        PAST COMMENT CONFIRMATION         │
├─────────────────────────────────────────┤        1001
│                                          │
│  CONTENT: BARCODE UNREADABLE             │
│                                          │
│  HANDLING METHOD:                        │
│  PLEASE CONFIRM BARCODE                  │
│                                          │
│  ALARM GENERATION DEVICE:                │
│  PRE-PROCESSING DEVICE 1 (POSITION 1B)   │
│                                          │
│  HANDLING STOP POSITION:                 │
│  NONE (NONE)                             │
│                                          │
│  PAST COMMENT                            │
│  ┌────────────────────────────────────┐  │
│  │ RUBBING OF BARCODE OCCURRED        │  │
│  │ (TARO TAKAHASHI, 06/10/2018-09:05:30)│ │
│  │                                    │  │
│  │                                    │  │
│  └────────────────────────────────────┘  │
│                                          │
│              ┌──────────┐                │
│              │  RETURN  │                │
│              └──────────┘                │
└─────────────────────────────────────────┘
                    1002
```

[FIG. 11]

1101

START — 1102

DETECT ALARM — 1103

ACQUIRE LIST OF PERSON-IN-CHARGE WHO CAN HANDLE ALARM — 1104

DETERMINE ALARM NOTIFICATION ORDER — 1105

WHETHER THERE IS PERSON-IN-CHARGE CANDIDATE — 1106    NO

YES

ALARM NOTIFICATION — 1107

TIME-OUT    DETERMINE TIME-OUT — 1108

NO TIME-OUT

CANNOT BE HANDLED    ACQUIRE PERSON-IN-CHARGE NOTIFICATION ANSWER — 1109

CAN BE HANDLED

UPDATE ALARM INFORMATION MANAGEMENT TABLE (ALARM HANDLING PERSON-IN-CHARGE) — 1110

UPDATE ALARM INFORMATION MANAGEMENT TABLE (REGISTER NEW ALARM) — 1113

CANNOT BE HANDLED    ACQUIRE PERSON-IN-CHARGE HANDLING ANSWER — 1111

NOTIFY MANAGER — 1115

COMPLETE HANDLING

EXCLUDE NOTIFIER FROM LIST OF PERSON-IN-CHARGE WHO CAN HANDLE ALARM — 1114

UPDATE ALARM INFORMATION MANAGEMENT TABLE (COMPLETION FLAG) — 1112

END — 1116

[FIG. 12]

1201

```
                              ┌────────────────────┐
                              │       START        │────── 1202
                              └────────────────────┘
                                        │
                                        ▼
            NO          ◇ WHETHER                    ◇────── 1203
      ┌─────────────────  THERE IS PERSON-IN-CHARGE WHO
      │                   CAN HANDLE ALARM
      │                           │
      │                          YES
      │                           ▼
      │                 ┌────────────────────┐
      │                 │ CALCULATE PERSON-IN-CHARGE │──── 1204
      │                 │   SCHEDULE SCORE    │
      │                 └────────────────────┘
      │                           │
      │                           ▼
      │                 ┌────────────────────┐
      │                 │ CALCULATE PERSON-IN-CHARGE │──── 1205
      │                 │ POSITION INFORMATION SCORE │
      │                 └────────────────────┘
      │                           │
      │                           ▼
      │                 ┌────────────────────┐
      │                 │ CALCULATE PRIORITY SCORE OF │─── 1206
      │                 │   PERSON-IN-CHARGE   │
      │                 │ WHO CAN HANDLE ALARM │
      │                 └────────────────────┘
      │                           │
      │                           ▼
      │                 ┌────────────────────┐
      │                 │ SORT LIST OF PERSON-IN-CHARGE │── 1207
      │                 │ WHO CAN HANDLE ALARM │
      │                 └────────────────────┘
      │                           │
      └───────────────────────────┤
                                  ▼
                        ┌────────────────────┐
                        │        END         │──── 1208
                        └────────────────────┘
```

[FIG. 13]

212

| ALARM MANAGEMENT NUMBER | ALARM GENERATION DATE AND TIME | ALARM CODE | ALARM GENERATION LOCATION | ALARM GENERATION DEVICE NAME | ALARM CONTENT | ALARM HANDLING METHOD | ALARM HANDLING PERSON-IN-CHARGE | COMMENT | RELATED ALARM MANAGEMENT NUMBER | COMPLETION FLAG |
|---|---|---|---|---|---|---|---|---|---|---|
| 302 | 303 | 304 | 305 | 306 | 307 | 308 | 309 | 310 | 311 | 312 |
| 20180610-0001 | 06/10/2018 08:30:20 | 1101 | 3B | ANALYZER 1 | PREPARATION FAILURE BEFORE ANALYSIS | PLEASE CONFIRM DEVICE | NULL | NULL | NULL | 0 |
| : | : | : | : | | : | : | : | : | : | : |

1301

[FIG. 14]

<u>214</u>

| TASK ID | PERSON-IN-CHARGE MANAGEMENT NUMBER | WORK CONTENT | WORK DIFFICULTY | TASK START TIME | COMPLETION FLAG |
|---|---|---|---|---|---|
| : | : | : | : | : | : |
| 001 | P001 | SAMPLE ACCEPTANCE | 5 | 06/10/2018-08:20:00 | 0 |
| 005 | P002 | SAMPLE ACCEPTANCE | 5 | 06/10/2018-08:25:00 | 0 |
| 009 | P003 | ANALYSIS PREPARATION | 2 | 06/10/2018-08:27:20 | 0 |
| 013 | P004 | DEVICE INSPECTION | 3 | 06/10/2018-08:00:10 | 0 |
| : | : | : | : | : | : |

502 503 504 505 506 507

EP 4 012 417 B1

[FIG. 15]

212

| | 302 | 303 | 304 | 305 | 306 | 307 | 308 | 309 | 310 | 311 | 312 |
| ALARM MANAGEMENT NUMBER | ALARM GENERATION DATE AND TIME | ALARM CODE | ALARM GENERATION LOCATION | ALARM GENERATION DEVICE NAME | ALARM CONTENT | ALARM HANDLING METHOD | ALARM HANDLING PERSON-IN-CHARGE | COMMENT | RELATED ALARM MANAGEMENT NUMBER | COMPLETION FLAG |
|---|---|---|---|---|---|---|---|---|---|---|
| 20180610-0003 | 06/10/2018 - 09:52:40 | 1201 | 3D | ANALYZER 5 | ZERO ALB REAGENT REMAINING | PLEASE REPLACE ALB REAGENT | NULL | NULL | NULL | 0 |
| : | : | : | : | : | : | : | : | : | : | : |

1501

[FIG. 16]

214

| TASK ID | PERSON-IN-CHARGE MANAGEMENT NUMBER | WORK CONTENT | WORK DIFFICULTY | TASK START TIME | COMPLETION FLAG |
|---|---|---|---|---|---|
| : | : | : | : | : | : |
| 011 | P001 | SAMPLE ACCEPTANCE | 5 | 06/10/2018-09:20:00 | 0 |
| 016 | P002 | ANALYSIS PREPARATION | 2 | 06/10/2018-09:25:00 | 0 |
| 022 | P003 | ANALYSIS PREPARATION | 2 | 06/10/2018-09:27:20 | 0 |
| 023 | P004 | DEVICE INSPECTION | 3 | 06/10/2018-09:00:10 | 0 |
| : | : | : | : | : | : |

502 503 504 505 506 507

[FIG. 17]

214

| TASK ID | PERSON-IN-CHARGE MANAGEMENT NUMBER | WORK CONTENT | WORK DIFFICULTY | TASK START TIME | COMPLETION FLAG |
|---|---|---|---|---|---|
| : | : | : | : | : | : |
| 011 | P001 | SAMPLE ACCEPTANCE | 5 | 06/10/2018-09:20:00 | 0 |
| 017 | P003 | ANALYSIS PREPARATION | 2 | 06/10/2018-09:25:00 | 2 |
| 032 | P003 | ALARM HANDLING | 3 | 06/10/2018-09:53:00 | 0 |
| 024 | P002 | ANALYSIS PREPARATION | 2 | 06/10/2018-09:27:20 | 0 |
| 025 | P004 | DEVICE INSPECTION | 3 | 05/10/2018-09:00:10 | 0 |
| : | : | : | : | : | : |

502 503 504 505 506 507

1701

1702

[FIG. 18]

1801

| ALARM MANAGEMENT NUMBER | ALARM GENERATION DATE AND TIME | ALARM CODE | ALARM SUB-CODE | ALARM GENERATION LOCATION | ALARM GENERATION DEVICE NAME | ALARM CONTENT | ALARM HANDLING METHOD | ALARM HANDLING PERSON-IN-CHARGE | COMMENT | RELATED ALARM MANAGEMENT NUMBER | COMPLETION FLAG |
|---|---|---|---|---|---|---|---|---|---|---|---|
| 20180610-0005 | 06/10/2018-10:01:13 | 1401 | 01 | 3D | ANALYZER 5 | FULL SAMPLE AFTER ANALYSIS | PLEASE REMOVE SAMPLE AFTER ANALYSIS | NULL | NULL | NULL | 0 |
| 20180610-0005 | 06/10/2018-10:01:13 | 1401 | 02 | 3D | ANALYZER 5 | FULL SAMPLE AFTER ANALYSIS | PLEASE REMOVE SAMPLE AFTER ANALYSIS | NULL | NULL | NULL | 0 |
| : | : | : | : | : | : | : | : | : | : | : | : |

302  303  304  1802  305  306  307  308  309  310  311  312

1810  1811

[FIG. 19]

213

| PERSON-IN-CHARGE MANAGEMENT NUMBER | PERSON-IN-CHARGE NAME | ATTENDANCE STATUS | ALARM THAT CAN BE HANDLED |
|---|---|---|---|
| P001 | HANAKO SATO | 1 | 1101, 1201, 1301, 1401-01, 1401-02, 2101, ⋯ |
| P002 | TARO SUZUKI | 1 | 1101, 1201, 1301, 1401-01, ⋯ |
| P003 | TARO TAKAHASHI | 1 | 1101, 1201, 1401-01, 1401-02, 2101, ⋯ |
| P004 | JIRO TANAKA | 1 | 1401-02, 2101, ⋯ |
| P005 | EIKO ITO | 0 | ⋯ |
| ⋮ | ⋮ | ⋮ | ⋮ |

402   403   404   405

[FIG. 20]

2001

| ALARM CODE | ALARM SUB-CODE | ALARM EXPLANATION | ALARM HANDLING METHOD | ALARM HANDLING STOP POSITION | ALARM HANDLING STOP POSITION NAME | NUMBER OF WORKER FOR ALARM HANDING |
|---|---|---|---|---|---|---|
| 1101 | NULL | PREPARATION FAILURE BEFORE ANALYSIS | PLEASE CONFIRM DEVICE | NULL | NULL | 1 |
| 2101 | NULL | BARCODE UNREADABLE | PLEASE CONFIRM BARCODE | NULL | NULL | 1 |
| 1201 | NULL | ZERO ALB REAGENT REMAINING | PLEASE REPLACE ALB REAGENT | 7B | REAGENT STORAGE | 1 |
| 1301 | NULL | SAMPLE CUP SHORTAGE | PLEASE ADD SAMPLE CUP | 11B | CONSUMABLE STORAGE | 1 |
| 1401 | 01 | FULL SAMPLE AFTER ANALYSIS | PLEASE REMOVE SAMPLE AFTER ANALYSIS | NULL | NULL | 2 |
| | 02 | | | 9D | WORK TABLE | |
| : | : | : | : | : | : | : |

602   2002   603   604   605   606   2003

[FIG. 21]

REGISTER ALARM THAT CAN BE HANDLED 2101

PLEASE ANSWER QUESTION

Q2. NUMBER OF YEARS IN SERVICE?

2102 ○ WITHIN 5 YEARS

2103 ○ 3 YEARS TO 10 YEARS

2104 ○ MORE THAN 10 YEARS

CHECK OPTIONS AND CLICK "NEXT"

| NEXT | RETURN |

2105 2106

[FIG. 22]

216

| QUESTION NO | QUESTION CONTENT | ANSWER ID | ANSWER CONTENT | ALARM THAT CAN BE HANDLED |
|---|---|---|---|---|
| ⋮ | ⋮ | ⋮ | ⋮ | ⋮ |
| 2 | NUMBER OF YEARS IN SERVICE? | 0 | WITHIN 5 YEARS | 1101, ⋯ |
| 2 | NUMBER OF YEARS IN SERVICE? | 1 | 5 YEARS TO 10 YEARS | 1101, 1301, ⋯ |
| 2 | NUMBER OF YEARS IN SERVICE? | 2 | MORE THAN 10 YEARS | 1101, 1201, 1301, 1401, 2101, ⋯ |
| ⋮ | ⋮ | ⋮ | ⋮ | ⋮ |

2202 2203 2204 2205 2206

[FIG. 23]

2301

START — 2302

START TO REGISTER ALARM THAT CAN BE HANDLED — 2303

WHETHER THERE IS QUESTION — 2304

NO

YES

DISPLAY QUESTION ON TERMINAL — 2305

ACQUIRE ANSWER TO QUESTION OF PERSON-IN-CHARGE — 2306

UPDATE PERSON-IN-CHARGE INFORMATION MANAGEMENT TABLE (ALARM THAT CAN BE HANDLED) — 2307

MOVE TO NEXT QUESTION — 2308

END — 2309

39

[FIG. 24]

2401

| CONFIRM ALL ALARMS |
|---|

TAP THE LIST AND CHECK THE ALARM
IN DETAIL.

| ALARM LIST |
|---|

2402

BARCODE UNREADABLE

GENERATION LOCATION:
PRE-PROCESSING DEVICE 1    09:05

TARO TAKAHASHI IS HANDLING

2403

ZERO ALB REAGENT REMAINING

GENERATION LOCATION:
ANALYZER 5    09:52

TARO SUZUKI IS HANDLING

2404

SAMPLE CUP SHORTAGE

GENERATION LOCATION:
ANALYZER 3    09:56

UNHANDLED

2405

FULL SAMPLE AFTER ANALYSIS

GENERATION LOCATION:
ANALYZER 5    10:01

UNHANDLED

:

**EP 4 012 417 B1**

**REFERENCES CITED IN THE DESCRIPTION**

**Patent documents cited in the description**

- WO 2013065528 A
- EP 2775307 A1
- JP 2018160808 A
- US 20190079107 A1